(19) Europäisches Patentamt
European Patent Office
Office européen des brevets

(11) EP 2 168 965 A1

(12) EUROPEAN PATENT APPLICATION

(43) Date of publication:
**31.03.2010 Bulletin 2010/13**

(51) Int Cl.:
***C07D 471/04*** (2006.01) ***A61K 31/437*** (2006.01)
***A61P 3/00*** (2006.01)

(21) Application number: **08016887.5**

(22) Date of filing: **25.09.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Santhera Pharmaceuticals (Schweiz) AG**
**4410 Liestal (CH)**

(72) Inventors:
- **Terinek, Miroslav**
  **4416 Bubendorf (CH)**
- **Nordhoff, Sonja**
  **4144 Arlesheim (CH)**
- **Soeberdt, Michael**
  **79618 Rheinfelden (DE)**
- **Rummey, Christian**
  **4053 Basel (CH)**
- **Feurer, Achim**
  **79424 Auggen (DE)**
- **Weyermann, Philipp**
  **4450 Sissach (CH)**

(74) Representative: **Grünecker, Kinkeldey, Stockmair & Schwanhäusser**
**Anwaltssozietät**
**Leopoldstrasse 4**
**80802 München (DE)**

(54) **Substituted imidazopyridine, imidazopyrazine, imidazopyridazine and imidazopyrimidine derivatives as melanocortin-4 receptor antagonists**

(57) The present invention relates to substituted imidazopyridine derivatives, imidazopyrazine derivatives, imidazopyridazine derivatives and imidazopyrimidine derivatives according to formula (I) as melanocortin-4 receptor (MC-4R) modulators, in particular as melanocortin-4 receptor antagonists. The antagonists are useful for the treatment of disorders and diseases such as cancer cachexia, muscle wasting, anorexia, emesis, amyotrophic lateral sclerosis (ALS), anxiety and depression.

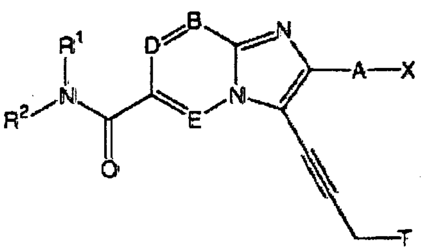

(I)

EP 2 168 965 A1

## Description

### Field of the Invention

The present invention relates to substituted imidazopyridine derivatives, imidazopyrazine derivatives, imidazopyridazine derivatives and imidazopyrimidine derivatives as melanocortin-4 receptor modulators. Depending on the structure and the stereochemistry, melanocortin-4 receptor modulators are either agonists or antagonists. The compounds of the invention are selective antagonists of the human melanocortin-4 receptor (MC-4R). The antagonists are useful for the treatment of disorders and diseases such as cancer cachexia, muscle wasting, anorexia, emesis, amytrophic lateral sclerosis (ALS), anxiety and depression.

### Background of the Invention

Melanocortins (MCs) stem from pro-opiomelanocortin (POMC) via proteolytic cleavage. These peptides, adrenocorticotropic hormone (ACTH), α-melanocyte-stimulating hormone (α-MSH), β-MSH and γ-MSH, range in size from 12 to 39 amino acids. The most important endogenous agonist for central MC-4R activation appears to be the tridecapeptide α-MSH. Among MCs, it was reported that α-MSH acts as a neurotransmitter or neuromodulator in the brain. MC peptides, particularly α-MSH, have a wide range of effects on biological functions including feeding behavior, pigmentation and exocrine function. The biological effects of α-MSH are mediated by a sub-family of 7-transmembrane G-protein-coupled receptors, termed melanocortin receptors (MC-Rs). Activation of any of these MC-Rs results in stimulation of cAMP formation.

To date, five distinct types of receptor subtype for MC (MC-1R to MC-5R) have been identified and these are expressed in different tissues.

MC-1 R was first found in melanocytes. Naturally occurring inactive variants of MC-1 R in animals were shown to lead to alterations in pigmentation and a subsequent lighter coat color by controlling the conversion of phaeomelanin to eumelanin through the control of tyrosinase. From these and other studies, it is evident that MC-1R is an important regulator of melanin production and coat color in animals and skin color in humans.

The MC-2R is expressed in the adrenal gland representing the ACTH receptor. The MC-2R is not a receptor for α-MSH but is the receptor for the adrenocorticotropic hormone I (ACTH I).

The MC-3R is expressed in the brain (predominately located in the hypothalamus) and peripheral tissues like gut and placenta, and knock-out studies have revealed that the MC-3R may be responsible for alterations in feeding behavior, body weight and thermogenesis.

The MC-4R is primarily expressed in the brain. Overwhelming data support the role of MC-4R in energy homeostasis. Genetic knock-outs and pharmacologic manipulation of MC-4R in animals have shown that agonizing the MC-4R causes weight loss and antagonizing the MC-4R produces weight gain (A. Kask, et al., "Selective antagonist for the melanocortin-4 receptor (HS014) increases food intake in free-feeding rats," Biochem. Biophys. Res. Commun., 245: 90-93 (1998)).

MC-5R is ubiquitously expressed in many peripheral tissues including white fat, placenta and a low level of expression is also observed in the brain. However its expression is greatest in exocrine glands. Genetic knock-out of this receptor in mice results in altered regulation of exocrine gland function, leading to changes in water repulsion and thermoregulation. MC-5R knockout mice also reveal reduced sebaceous gland lipid production (Chen et al., Cell, 91: 789-798 (1997)).

Attention has been focused on the study of MC-3R and MC-4R modulators and their use in treating body weight disorders, such as obesity and anorexia. However, evidence has shown that the MC peptides have potent physiological effects besides their role in regulating pigmentation, feeding behavior and exocrine function. In particular, α-MSH recently has been shown to induce a potent anti-inflammatory effect in both acute and chronic models of inflammation including inflammatory bowel-disease, renal ischemia/reperfusion injury and endotoxin-induced hepatitis. Administration of α-MSH in these models results in substantial reduction of inflammation-mediated tissue damage, a significant decrease in leukocyte infiltration and a dramatic reduction in elevated levels of cytokines and other mediators to near baseline levels. Recent studies have demonstrated that the anti-inflammatory actions of α-MSH are mediated by MC-1R. The mechanism by which agonism of MC-1R results in an anti-inflammatory response is likely through inhibition of the pro-inflammatory transcription activator, NF-κB. NF-κB is a pivotal component of the pro-inflammatory cascade, and its activation is a central event in initiating many inflammatory diseases. Additionally, anti-inflammatory actions of α-MSH may be, in part, mediated by agonism of MC-3R and/or MC-5R.

A specific single MC-R that may be targeted for the control of obesity has not yet been identified, although evidence has been presented that MC-4R signaling is important in mediating feeding behavior (S.Q. Giraudo et al., "Feeding effects of hypothalamic injection of melanocortin-4 receptor ligands," Brain Research, 80: 302-306 (1998)). Further evidence for the involvement of MC-Rs in obesity includes: 1) the agouti ($A^{vy}$) mouse which ectopically expresses

an antagonist of the MC-1 R, MC-3R and MC-4R is obese, indicating that blocking the action of these three MC-R's can lead to hyperphagia and metabolic disorders; 2) MC-4R knockout mice (D. Huszar et al., Cell, 88: 131-141 (1997)) recapitulate the phenotype of the agouti mouse and these mice are obese; 3) the cyclic heptapeptide melanotanin II (MT-II) (a non-selective MC-1R, -3R, -4R, and -5R agonist) injected intracerebroventricularly (ICV) in rodents, reduces food intake in several animal feeding models (NPY, ob/ob, agouti, fasted) while ICV injected SHU-9119 (MC-3R and 4R antagonist; MC-1 R and -5R agonist) reverses this effect and can induce hyperphagia; 4) chronic intraperitoneal treatment of Zucker fatty rats with an $\alpha$-NDP-MSH derivative (HP-228) has been reported to activate MC-1 R, -3R, -4R, and -5R and to attenuate food intake and body weight gain over a 12 week period (I. Corcos et al., "HP-228 is a potent agonist of melanocortin receptor-4 and significantly attenuates obesity and diabetes in Zucker fatty rats", Society for Neuroscience Abstracts, 23: 673 (1997)).

MC-4R appears to play a role in other physiological functions as well, namely controlling grooming behavior, erection and blood pressure. Erectile dysfunction denotes the medical condition of inability to achieve penile erection sufficient for successful intercourse. The term "impotence" is often employed to describe this prevalent condition. Synthetic melanocortin receptor agonists have been found to initiate erections in men with psychogenic erectile dysfunction (H. Wessells et al., "Synthetic Melanotropic Peptide Initiates Erections in Men With Psychogenic Erectile Dysfunction: Double-Blind, Placebo Controlled Crossover Study," J. Urol., 160: 389-393, 1998). Activation of melanocortin receptors of the brain appears to cause normal stimulation of sexual arousal. Evidence for the involvement of MC-R in male and/or female sexual dysfunction is detailed in WO 00/74679.

Diabetes is a disease in which a mammal's ability to regulate glucose levels in the blood is impaired because the mammal has a reduced ability to convert glucose to glycogen for storage in muscle and liver cells. In Type I diabetes, this reduced ability to store glucose is caused by reduced insulin production. "Type II diabetes" or "Non-Insulin Dependent Diabetes Mellitus" (NIDDM) is the form of diabetes which is due to a profound resistance to insulin stimulating or regulatory effect on glucose and lipid metabolism in the main insulin-sensitive tissues, muscle, liver and adipose tissue. This resistance to insulin responsiveness results in insufficient insulin activation of glucose uptake, oxidation and storage in muscle, and inadequate insulin repression of lipolysis in adipose tissue and of glucose production and secretion in liver. When these cells become desensitized to insulin, the body tries to compensate by producing abnormally high levels of insulin and hyperinsulemia results. Hyperinsulemia is associated with hypertension and elevated body weight. Since insulin is involved in promoting the cellular uptake of glucose, amino acids and triglycerides from the blood by insulin sensitive cells, insulin insensitivity can result in elevated levels of triglycerides and LDL which are risk factors in cardiovascular diseases. The constellation of symptoms which includes hyperinsulemia combined with hypertension, elevated body weight, elevated triglycerides and elevated LDL, is known as Syndrome X. MC-4R agonists might be useful in the treatment of NIDDM and Syndrome X.

Among MC receptor subtypes, the MC4 receptor is also of interest in terms of the relationship to stress and the regulation of emotional behavior, as based on the following findings. Stress initiates a complex cascade of responses that include endocrine, biochemical and behavioral events. Many of these responses are initiated by release of corticotropin-releasing factor (CRF) (Owen MJ and Nemeroff CB (1991) Physiology and pharmacology of corticotrophin releasing factor. Pharmacol Rev 43: 425-473). In addition to activation of the brain CRF system, there are several lines of evidence that melanocortins (MCs), which stem from proopiomelanocortin by enzymatic processing, mediate important behavioral and biochemical responses to stress and, consequently, stress-induced disorders like anxiety and depression (Anxiolytic-Like and Antidepressant-Like Activities of MCL0129 (1-[(*S*)-2-(4-Fluorophenyl)-2-(4-isopropylpiperadin-1-yl) ethyl]-4-[4-(2-methoxynaphthalen-1-yl)butyl]piperazine), a Novel and Potent Nonpeptide Antagonist of the Melanocortin-4 Receptor; Shigeyuki Chaki et al, J. Pharm. Exp. Ther. (2003) 304(2), 818-26).

Chronic diseases, such as malignant tumors or infections, are frequently associated with cachexia resulting from a combination of a decrease in appetite and a loss of lean body mass. Extensive loss of lean body mass is often triggered by an inflammatory process and is usually associated with increased plasma levels of cytokines (e.g. TNF-$\alpha$), which increase the production of $\alpha$-MSH in the brain. Activation of MC4 receptors in the hypothalamus by $\alpha$-MSH reduces appetite and increases energy expenditure. Experimental evidence in tumor bearing mice suggests that cachexia can be prevented or reversed by genetic MC4 receptor knockout or MC4 receptor blockade. The increased body weight in the treated mice is attributable to a larger amount of lean body mass, which mainly consists of skeletal muscle (Marks D.L. et al. Role of the central melanocortin system in cachexia. Cancer Res. (2001) 61: 1432-1438).

Internal studies in ferrets have shown that a pharmacological inhibition of melanocortin-4 receptors strongly inhibits emesis. Accordingly, MC-4R inhibitors could be used to treat emesis, especially in patients undergoing chemotherapy.

Clinical observations indicate, that progression of amyotrophic lateral sclerosis (ALS) might be inversely correlated with body weight (e.g. Ludolph AC, Neuromuscul Disord. (2006) 16 (8):530-8). Accordingly, MC-4R inhibitors could be used to treat ALS patients.

Melanocortin-4 receptor modulators have been previously described in the literature. For example, substituted phenylpiperidine derivatives have been synthesized and explored as MC-4R agonists as well as antagonists.

In view of the unresolved deficiencies in treatment of various diseases and disorders as discussed above, it is an object of the present invention to provide novel compounds with improved ability to cross the blood brain barrier, which are useful as melanocortin-4 receptor antagonists to treat cancer cachexia, muscle wasting, anorexia, amyotrophic lateral sclerosis (ALS), emesis, anxiety, depression, and other diseases with MC-4R involvement.
Surprisingly, it has been found that novel imidazopyridine derivatives, imidazopyrazine derivatives, imidazopyridazine derivatives and imidazopyrimidine derivatives according to formula (I) shown below solve the object of the present invention.

## Summary of the Invention

The present invention relates to substituted imidazopyridine derivatives, imidazopyrazine derivatives, imidazopyridazine derivatives and imidazopyrimidine derivatives of structural formula (I)

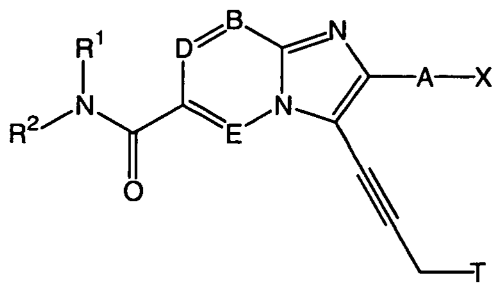

(I)

wherein $R^1$, $R^2$, A, B, D, E, T and X are defined as described below.
The imidazopyridine, imidazopyrazine, imidazopyridazine and imidazopyrimidine derivatives of structural formula (I) are effective as melanocortin receptor modulators and are particularly effective as selective melanocortin-4 receptor (MC-4R) antagonists. They are therefore useful for the treatment of disorders where the inactivation of the MC-4R is involved. The antagonists are useful for the treatment of disorders and diseases such as cancer cachexia, muscle wasting, anorexia, emesis, amyotrophic lateral sclerosis, anxiety and depression.
Thus, the present inventions relates to compounds of formula (I) for the treatment and/or prophylaxis of cancer cachexia, muscle wasting, anorexia, emesis, amyotrophic lateral sclerosis (ALS), anxiety and depression.
In a further aspect, the invention relates to the use of a compound of formula (I) for the preparation of a medicament for the treatment and/or prophylaxis of disorders, diseases or conditions responsive to the inactivation of the melanocortin-4 receptor in a mammal, such as cancer cachexia, muscle wasting, anorexia, amyotrophic lateral sclerosis (ALS), anxiety and depression.
The present invention also relates to pharmaceutical compositions comprising the compounds of the present invention and a pharmaceutically acceptable carrier.

## Detailed Description of the Invention

The present invention relates to substituted imidazopyridine derivatives, imidazopyrazine derivatives, imidazopyridazine derivatives and imidazopyrimidine derivatives useful as melanocortin receptor modulators. The compounds of the present invention are in particular useful as selective MC-4R antagonists.
Substituted *N*-benzyl-*N*-methyl-2-phenyl-5-diethylamido-3-methylamino-imidazo[1,2-*a*] pyridines are known from WO-A-02/066478 which describes antagonists of gonadotropin releasing hormone. The present invention relates to novel imidazopyridines, imidazopyrazines, imidazopyridazines and imidazopyrimidines which are used as antagonists of MC-4R.
The compounds of the present invention are represented by structural formula (I)

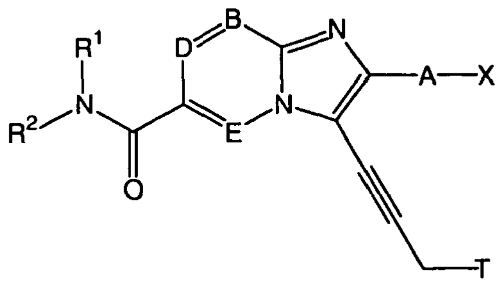

(I)

and enantiomers, diastereomers, tautomers, solvates and pharmaceutically acceptable salts thereof, wherein

$R^1$ and $R^2$ are independently from each other selected from

H,
$C_{1-6}$ alkyl,
$C_{1-6}$ alkylene-O-$C_{1-6}$alkyl
$C_{1-3}$ alkylene-heterocyclyl, and
$C_{1-6}$ alkylene-$C_{3-7}$cycloalkyl, or

$R^1$ and $R^2$, together with the nitrogen atom to which they are attached to, form a 5 to 6-membered ring which may additionally contain one oxygen atom in the ring and which is unsubstituted or substituted by one or more substituents selected from OH, $C_{1-6}$alkyl, O-$C_{1-6}$alkyl, $C_{0-3}$alkylene-$C_{3-5}$cycloalkyl, $C_{1-6}$alkylene-O-$C_{1-6}$alkyl and $(CH_2)_{0-3}$ -phenyl;
B, D and E are independently from each other selected from CH and N;
A is -NH-,

-N($C_{1-6}$alkyl)-,
-$C_{1-6}$alkylene,
-$C_{2-6}$alkenylene,
-$C_{2-6}$alkinylene, or
a bond
wherein alkylene, alkenylene and alkinylene are unsubstituted or substituted with one ore more $R^6$;

$R^6$ is independently selected from

$C_{1-6}$alkyl,
$OR^{13}$,
$NR^{14a}R^{14b}$,
halogen,
phenyl, and
heteroaryl,
wherein phenyl and heteroaryl are unsubstituted or substituted by 1 to 3 $R^{3a}$; X is H,
CN,
$C_{3-8}$cycloalkyl, unsubstituted or substituted with one or more halogen atoms, phenyl,
phenyl which is fused with

(a) a saturated or unsaturated heterocyclic 5 to 6-membered ring containing 1 or 2 heteroatoms independently selected from O and N, or
(b) a 5 to 6-membered heteroaryl containing1 to 3 heteroatoms independently selected from O and N,

4 to 8-membered saturated or unsaturated heterocyclyl containing 1 to 4 heteroatoms independently selected from N, O and S,
5- to 6-membered heteroaryl containing 1 to 4 heteroatoms independently selected from N, O and S,
5- to 6-membered heteroaryl containing 1 to 4 heteroatoms independently selected from N, O and S, fused with

(a) 5 to 6-membered heteroaryl containing 1 to 4 heteroatoms independently selected from N, O and S, or
(b) a saturated or unsaturated heterocyclic 5 to 6-membered ring containing1 or 2 heteroatoms independently selected from O and N,

-C(O)-$R^5$,
-$OR^{13}$,
halogen or
$NR^{14a}R^{14b}$,
wherein each phenyl, heterocyclyl or heteroaryl is unsubstituted or substituted by 1 to 3 $R^{3a}$ and/or 1 $R^{3b}$ and/or 1 $R^4$;

$R^{3a}$ is independently selected from

halogen,
CN,
$C_{1-6}$alkyl, unsubstituted or substituted with one or more substituents independently selected from halogen, OH, O-$C_{1-6}$alkyl and 5 to 6-membered heterocyclyl containing 1 to 2 heteroatoms independently selected from N, O and S,
O-$C_{1-6}$alkyl, wherein alkyl is unsubstituted or substituted with one or more halogen atoms, and
OH;

$R^{3b}$ is C(O)-$C_{1-6}$alkyl,

C(O)O-$C_{1-6}$alkyl,
C(O)$NH_2$,
C(O)NH-$C_{1-6}$alkyl,
C(O)N-($C_{1-6}$alkyl)$_2$,
$SO_2$-$C_{1-6}$alkyl,
C(O)NH-$SO_2$-$C_{1-6}$alkyl,
oxo, whereby the ring is at least partially saturated,
$NH_2$,
NH-$C_{1-6}$alkyl, wherein alkyl is unsubstituted or substituted by OH or
O-$C_{1-6}$ alkyl,
N-($C_{1-6}$alkyl)$_2$, wherein each alkyl is unsubstituted or substituted by OH or O-$C_{1-6}$ alkyl,
NH-$SO_2$-$CH_3$, or
NH-$SO_2$-$CF_3$;

$R^4$ is 5 to 6-membered saturated or unsaturated heterocyclyl containing 1 to 3

heteroatoms independently selected from N, O and S, or
5 to 6-membered heteroaryl containing 1 to 3 heteroatoms independently selected from N, O and S,
wherein each heterocyclyl and heteroaryl is unsubstituted or substituted by 1 or 2 $R^{3a}$.

$R^5$ is H,

OH,
$C_{1-6}$alkyl, unsubstituted or substituted with one or more halogen atoms,
O-$C_{1-6}$alkyl, wherein alkyl is unsubstituted or substituted with one or more $R^{15}$, phenyl,
4 to 8-membered saturated or unsaturated heterocyclyl containing 1 to 3 heteroatoms selected from N, O or S; or
$NR^{15a}R^{15b}$,
wherein each phenyl and heterocyclyl is unsubstituted or substituted by 1 to 3 $R^{3a}$ and/or 1 $R^4$;
T is

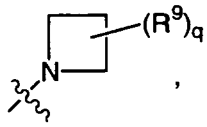,
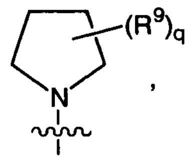,
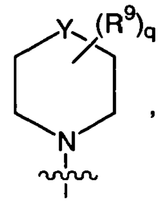,

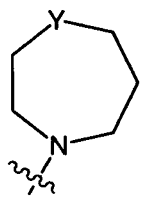,
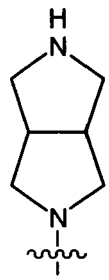,
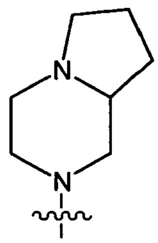,

or $NR^{11}R^{12}$;

$R^9$ is $NH_2$,

OH,
O-$C_{1-6}$alkyl, wherein alkyl is unsubstituted or substituted by 1 to 3 halogen atoms,
$C_{1-6}$alkyl, wherein alkyl is unsubstituted or substituted by 1 to 3 halogen atoms, halogen,
NH($C_{1-6}$alkyl),
N($C_{1-6}$alkyl)$_2$,
phenyl, or
heteroaryl,
wherein phenyl and heteroaryl are unsubstituted or substituted by 1 to 3 $R^{3a}$;

q is 0, 1 or 2;
Y is $CH_2$,

$NR^{10}$, or
O;

$R^{10}$ is H,

$C_{1-6}$alkyl, or
$(CH_2)_{0-6}$-$C_{3-7}$cycloalkyl;

$R^{11}$ and $R^{12}$ are independently from each other selected from

H,
$C_{1-6}$alkyl,
$(CH_2)_{0-2}$-$C_{3-7}$cycloalkyl and
$C_{1-6}$alkylene-O-$C_{1-6}$alkyl;
wherein alkyl, alkylene and cycloalkyl are unsubstituted or substituted by 1 to 3 $R^{3a}$;

$R^{13}$ is H ,

$C_{1-6}$alkyl, unsubstituted or substituted with one or more substituents selected from halogen,
phenyl, or
heteroaryl,
wherein phenyl and heteroaryl are unsubstituted or substituted by 1 to 3 $R^{3a}$;

$R^{14a}$ and $R^{14b}$ are independently from each other selected from

H,
$C_{1-6}$alkyl, unsubstituted or substituted with one or more substituents selected from halogen, OH, O($C_{1-6}$alkyl), $NH_2$, NH($C_{1-6}$alkyl) and N($C_{1-6}$alkyl)$_2$,
phenyl,
5 to 6-membered heteroaryl containing 1 to 4 heteroatoms independently selected from N, O and S,
wherein phenyl and heteroaryl are unsubstituted or substituted by 1 to 3 $R^{3a}$, and
C(O)$C_{1-6}$alkyl;

$R^{15}$, $R^{15a}$ and $R^{15b}$ are independently from each other selected from

H,
$C_{1-6}$alkyl, unsubstituted or substituted with one or more substituents selected from halogen, OH, O($C_{1-6}$alkyl), $NH_2$, NH($C_{1-6}$alkyl) and N($C_{1-6}$alkyl)$_2$,
$C_{0-3}$alkylene-$C_{3-5}$cycloalkyl,
phenyl and
5 to 6-membered heteroaryl containing 1 to 4 heteroatoms independently selected from N, O and S,
wherein phenyl and heteroaryl are unsubstituted or substituted by 1 to 3 $R^{3a}$.

In preferred embodiments of the present invention, the variants $R^1$, $R^2$, A, B, D, E, T and X of formula (I) independently from each other have the following meanings. Hence, one or more of the variants $R^1$, $R^2$, A, B, D, E, T and X can have the preferred or more preferred meanings given below with all combinations of the general and preferred definitions being subject of the invention.

The variants B, D and E may each represent a CH group or a nitrogen atom. In a preferred embodiment, one of B, D and E represents a nitrogen atom and the remaining two variants are simultaneously CH. More preferably, B, D and E each represent a CH group.

In a further preferred embodiment, the variant A represents -NH-, -N($C_{1-6}$alkyl)-, -$C_{2-6}$alkinylene or a bond. More preferably, A represents -NH-, -$C_{2-6}$alkinylene or a bond, most preferably a bond.

It is further preferred that $R^1$ and $R^2$ independently from each other represent $C_{3-6}$alkyl or $C_{1-6}$alkylene-$C_{3-7}$cycloalkyl or that $R^1$ and $R^2$ form together with the nitrogen atom to which they are attached a 5 to 6-membered ring which may additionally contain one oxygen atom in the ring and which is unsubstituted or substituted by one or more substituents selected from OH, $C_{1-6}$alkyl, $C_{0-3}$alkylene-$C_{3-5}$cycloalkyl, O-$C_{1-6}$alkyl, $C_{1-6}$alkylene-O-$C_{1-6}$alkyl and $(CH_2)_{0-3}$-phenyl. More preferably, $R^1$ and $R^2$ independently from each other represent $C_{3-6}$alkyl.

The variant T is preferably selected from

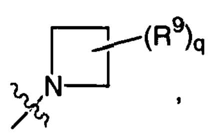 , 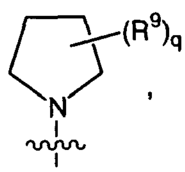 , 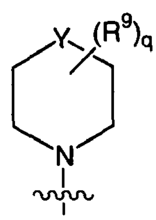

or

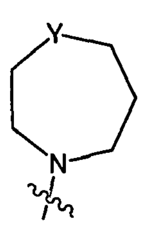 .

More preferably, T represents a pyrrolidine group.

In an alternatively preferred embodiment, the variant T is $NR^{12}R^{13}$. Therein, the variants $R^{12}$ and $R^{13}$ are preferably independently from each other selected from H, $C_{1-3}$alkyl or $(CH_2)_{0-2}$-$C_{3-6}$cycloalkyl, wherein alkyl and cycloalkyl are unsubstituted or substituted by 1 to 3 $R^{14}$, wherein $R^{14}$ is defined as above.

It is preferred that the variant Y is $CH_2$ or $NR^{10}$, wherein $R^{10}$ is defined as above.

Preferably, $R^9$ is $NH_2$, $C_{1-6}$alkyl, NH($C_{1-6}$alkyl) or N($C_{1-6}$alkyl)$_2$.

The index q is preferably 0 or 1.

Regarding the variant X, said variant preferably represents

phenyl,
phenyl which is fused with

(a) a saturated or unsaturated heterocyclic 5 to 6-membered ring, containing 1 or 2 heteroatoms independently selected from O and N, or
(b) a 5 to 6-membered heteroaryl containing1 to 3 heteroatoms independently selected from O and N,

4 to 8-membered saturated or unsaturated heterocyclyl containing 1 to 4 heteroatoms independently selected from N, O and S, or
5 to 6-membered heteroaryl containing 1 to 4 heteroatoms independently selected from N, O and S,
5 to 6-membered heteroaryl containing 1 to 4 heteroatoms independently selected from N, O and S, fused with

(a) 5 to 6-membered heteroaryl containing 1 to 4 heteroatoms independently selected from N, O and S,
(b) a saturated or unsaturated heterocyclic 5- or 6-membered ring containing1 or 2 heteroatoms independently selected from O and N,

wherein each phenyl, heterocyclyl or heteroaryl is unsubstituted or substituted by 1 to 3 $R^{3a}$ and/or 1 $R^{3b}$ and/or 1 $R^4$, wherein $R^{3a}$, $R^{3b}$ and $R^4$ are defined as above, or -C(O)-$R^5$.

In said preferred embodiment, the variant $R^5$ is preferably -O-$C_{1-6}$alkyl, wherein alkyl is unsubstituted or substituted with one or more $R^{15}$, wherein $R^{15}$ is defined as above.

More preferably, X represents $COR^5$, wherein $R^5$ is defined as above, phenyl, a 6-membered saturated or unsaturated heterocyclyl containing 1 or 2 heteroatoms independently selected from N and O a 5- to 6-membered heteroraryl containing 1 or 2 heteroatoms independently selected N and O or a 6-membered nitrogen-containing heteroaryl fused with 5- or 6-membered nitrogen-containing heteroaryl or fused with a saturated 5- or 6-membered heterocycle containing 1 or 2 heteroatoms independently selected from N and O wherein each phenyl, heterocyclyl or heteroaryl is unsubstituted or substituted by 1 to 3 $R^{3a}$ and/or 1 $R^{3b}$ and/or 1 $R^4$, wherein $R^{3a}$, $R^{3b}$ and $R^4$ are defined as above.

Compounds of the formula (I) in which some or all of the above-mentioned groups have the preferred or more preferred meanings are also an object of the present invention.

In the above and the following, the employed terms have the meaning as described below:

Alkyl is a straight chain or branched alkyl having 1, 2, 3, 4, 5 or 6 carbon atoms, such as methyl, ethyl, n-propyl, isopropyl, n-butyl, isobutyl, tert-butyl, n-pentyl, isopentyl, neopentyl, or hexyl.
Alkenyl is a straight chain or branched alkyl having 1, 2, 3, 4, 5 or 6 carbon atoms and one to three double bonds, preferably one or two double bonds, most preferably one double bound. Preferred examples of a $C_{2-6}$ alkenyl group are ethenyl, prop-1-enyl, prop-2-enyl, isoprop-1-enyl, n-but-1-enyl, n-but-2-enyl, n-but-3-enyl, isobut-1-enyl, isobut-2-enyl, n-pent-1-enyl, n-pent-2-enyl, n-pent-3-enyl, n-pent-4-enyl, n-pent-1,3-enyl, isopent-1-enyl, isopent-2-enyl, neopent-1-enyl, n-hex-1-enyl, n-hex-2-enyl, n-hex-3-enyl, n-hex-4-enyl, n-hex-5-enyl, n-hex-1,3-enyl, n-hex-2,4-enyl, n-hex-3,5-enyl, and n-hex-1,3,5-enyl. More preferred examples of a $C_{2-6}$ alkenyl group are ethenyl and prop-1-enyl.

Alkinyl is a straight chain or branched alkyl having 1, 2, 3, 4, 5 or 6 carbon atoms and one to three triple bonds, preferably one or two triple bonds, most preferably one triple bond. Preferred examples of a $C_{2-6}$alkinyl group are ethinyl, prop-1-inyl, prop-2-inyl, n-but-1-inyl, n-but-2-inyl, n-but-3-inyl, n-pent-1-inyl, n-pent-2-inyl, n-pent-3-inyl, n-pent-4-inyl, n-pent-1,3-inyl, isopent-1-inyl, neopent-1-inyl, n-hex-1-inyl, n-hex-2-inyl, n-hex-3-inyl, n-hex-4-inyl, n-hex-5-inyl, n-hex-1,3-inyl, n-hex-2,4-inyl, n-hex-3,5-inyl and n-hex-1,3,5-inyl. More preferred examples of a $C_{2-6}$alkinyl group are ethinyl and prop-1-inyl.

Cycloalkyl is an alkyl ring having preferably 3 to 7 carbon atoms at the most, such as cyclopropyl, cyclobutyl, cyclopentyl, cyclohexyl or cycloheptyl, more preferably having 3 to 6 carbon atoms.

Heteroaryl is a 5- or 6-membered aromatic cyclic moiety having 1 to 5 carbon atoms and at least one heteroatom independently selected from O, N and/or S and is preferably selected from thienyl, pyrrolyl, imidazolyl, pyrazolyl, pyridyl, pyrazinyl, pyrimidinyl, pyridazinyl, isothiazolyl, isoxazyl, furanyl, and indazolyl, more preferably from thienyl, furanyl, imidazlyl, pyridyl, and pyrimidinyl.

Heterocyclyl is a 3- to 8-membered saturated cycloalkane ring or unsaturated cycloalkene ring containing at least one heteroatom selected from O, N and/or S and 1 to 7 carbon atoms. Preferably, heterocyclyl is a 4 to 8-membered ring and is preferably selected from tetrahydrofuranyl, azetidinyl, pyrrolidinyl, piperidinyl, pyranyl, morpholinyl, thiomorpholinyl, more preferably from piperidinyl and pyrrolidinyl.

Halogen is a halogen atom selected from F, Cl, Br and I, preferably from F, Cl and Br.

The compounds of structural formula (I) are effective as melanocortin receptor modulators and are particularly effective as selective modulators of MC-4R. They are useful for the treatment and/or prevention of disorders responsive to the inactivation of MC-4R, such as cancer cachexia, muscle wasting, anorexia, emesis, amyotrophic lateral sclerosis, anxiety, depression and other diseases with MC-4R involvement.

## Optical Isomers - Diastereomers - Geometric Isomers - Tautomers

Compounds of structural formula (I) contain one or more asymmetric centers and can occur as racemates and racemic mixtures, single enantiomers, diastereomeric mixtures and individual diastereomers. The present invention is meant to comprehend all such isomeric forms of the compounds of structural formula (I).

Compounds of structural formula (I) may be separated into their individual diastereoisomers by, for example, fractional crystallization from a suitable solvent, for example methanol or ethyl acetate or a mixture thereof, or via chiral chromatography using an optically active stationary phase. Absolute stereochemistry may be determined by X-ray crystallography of crystalline products or crystalline intermediates which are derivatized, if necessary, with a reagent containing an asymmetric center of known absolute configuration.

Alternatively, any stereoisomer of a compound of the general formula (I) may be obtained by stereospecific synthesis using optically pure starting materials or reagents of known absolute configuration.

## Salts

The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, lithium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, formic, furnaric, gluconic, glutamic, hydrobromic, hydrochloric, isethionic, lactic, maleic, malic, mandelic, methanesulfonic, malonic, mucic, nitric, parnoic, pantothenic, phosphoric, propionic, succinic, sulfuric, tartaric, p-toluenesulfonic, trifluoroacetic acid and the like. Particularly preferred are citric, fumaric, hydrobromic, hydrochloric, maleic, phosphoric, sulfuric and tartaric acids.

It will be understood that, as used herein, references to the compounds of formula (I) are meant to also include the pharmaceutically acceptable salts.

## Utility

Compounds of formula (I) are melanocortin receptor antagonists and as such are useful in the treatment, control or prevention of diseases, disorders or conditions responsive to the inactivation of one or more of the melanocortin receptors including, but not limited to, MC-1R, MC-2R, MC-3R, MC-4R or MC-5R. Such diseases, disorders or conditions include, but are not limited to, cancer cachexia, muscle wasting, anorexia, emesis, amyotrophic lateral sclerosis, anxiety and depression.

The compounds of formula (I) can be further used in the treatment, control or prevention of diseases, disorders or conditions which are responsive to the inactivation of one or more melanocortin receptors including, but not limited to,

MC-1R, MC-2R, MC-3R, MC-4R or MC-5R. Such diseases, disorders or conditions include, but are not limited to, hypertension, hyperlipidemia, osteoarthritis, cancer, gall bladder disease, sleep apnea, compulsion, neuroses, insomnia/sleep disorder, substance abuse, pain, fever, inflammation, immune-modulation, rheumatoid arthritis, skin tanning, acne and other skin disorders, neuroprotective and cognitive and memory enhancement including the treatment of Alzheimer's disease.

## Administration and Dose Ranges

Any suitable route of administration may be employed for providing a mammal, especially a human with an effective dosage of a compound of the present invention. For example, oral, rectal, topical, parenteral, ocular, pulmonary, nasal and the like may be employed. Dosage forms include tablets, troches, dispersions, suspensions, solutions, capsules, creams, ointments, aerosols and the like. Preferably, compounds of formula (I) are administered orally or topically.

The effective dosage of active ingredient employed may vary depending on the particular compound employed, the mode of administration, the condition being treated and the severity of the condition being treated. Such dosage may be ascertained readily by a person skilled in the art.

When treating cancer cachexia, muscle wasting or anorexia generally satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

## Formulation

The compounds of formula (I) are preferably formulated into a dosage form prior to administration. Accordingly the present invention also includes a pharmaceutical composition comprising a compound of formula (I) and a suitable pharmaceutical carrier.

The present pharmaceutical compositions are prepared by known procedures using well-known and readily available ingredients. In making the formulations of the present invention, the active ingredient (a compound of formula (I)) is usually mixed with a carrier, or diluted by a carrier, or enclosed within a carrier, which may be in the form of a capsule, sachet, paper or other container. When the carrier serves as a diluent, it may be a solid, semisolid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosol (as a solid or in a liquid medium), soft and hard gelatin capsules, suppositories, sterile injectable solutions and sterile packaged powders.

Some examples of suitable carriers, excipients and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl and propylhydroxybenzoates, talc, magnesium stearate and mineral oil. The formulations can additionally include lubricating agents, wetting agents, emulsifying and suspending agents, preserving agents, sweetening agents or flavoring agents. The pharmaceutical compositions of the invention may be formulated so as to provide quick, sustained or delayed release of the active ingredient after administration to the patient.

## Preparation of Compounds of the Invention

The compounds of formula (I) when existing as a diastereomeric mixture, may be separated into diastereomeric pairs of enantiomers by fractional crystallization from a suitable solvent such as methanol, ethyl acetate or a mixture thereof. The pair of enantiomers thus obtained may be separated into individual stereoisomers by conventional means by using an optically active acid as a resolving agent. Alternatively, any enantiomer of a compound of the formula (I) may be obtained by stereospecific synthesis using optically pure starting materials or reagents of known configuration.

The compounds of formula (I) of the present invention can be prepared according to the procedures of the following Schemes and Examples, using appropriate materials and are further exemplified by the following specific examples. Moreover, by utilizing the procedures described herein, in conjunction with ordinary skills in the art, additional compounds of the present invention claimed herein can be readily prepared. The compounds illustrated in the examples are not, however, to be construed as forming the only genus that is considered as the invention. The Examples further illustrate details for the preparation of the compounds of the present invention. Those skilled in the art will readily understand that known variations of the conditions and processes of the following preparative procedures can be used to prepare these compounds. The instant compounds are generally isolated in the form of their pharmaceutically acceptable salts, such as those described previously. The free amine bases corresponding to the isolated salts can be

generated by neutralization with a suitable base, such as aqueous sodium hydrogencarbonate, sodium carbonate, sodium hydroxide and potassium hydroxide, and extraction of the liberated amine free base into an organic solvent followed by evaporation. The amine free base isolated in this manner can be further converted into another pharmaceutically acceptable salt by dissolution in an organic solvent followed by addition of the appropriate acid and subsequent evaporation, precipitation or crystallization. All temperatures are degrees Celsius.

In the schemes, preparations and examples below, various reagent symbols and abbreviations have the following meanings

| | |
|---|---|
| AcOH | acetic acid |
| DCM | dichloromethane |
| DIEA | ethyl-diisopropylam ine |
| DMF | N,N-dimethylformamide |
| EDCI | 1-(3-dimethylaminopropyl)-3-ethylcarbodiimide hydrochloride |
| $Et_2O$ | diethyl ether |
| HOBt | 1-hydroxybenzotriazole hydrate |
| h | hour(s) |
| MeCN | acetonitrile |
| MeLi | methyllithium |
| NMM | N-methylmorpholine |
| MW | molecular weight |
| NBS | N-bromosuccinimide |
| NIS | N-iodosuccinimide |
| $PdCl_2(PPh_3)_2$ | bis(triphenylphosphine)palladium(II) dichloride |
| $Pd_2dba_3$ | tris(dibenzylideneacetone)dipalladium(0) |
| $Pd(dppf)_2Cl_2$ | 1,1'-bis(diphenylphosphino)ferrocene-palladium(II)dichloride dichloromethane adduct |
| $Pd(PPh_3)_4$ | tetrakis(triphenylphosphine)palladium(0) |
| RT | room temperature |
| TEA | triethylamine |
| THF | tetrahydrofurane |
| $t_R$ (min) | HPLC retention time |
| Xantphos | 9,9-dimethyl-4,5-bis(di-tert-butylphosphino)xanthene |

## Reaction Scheme 1:

## Synthesis of 2-amino-pyridine-5-carboxylic acid amides, 2-amino-pyrimidine-5-carboxylic acid amides, 2-amino-pyrazine-5-carboxylic acid amides and 6-amino-pyridazine-3-carboxylic acid amides

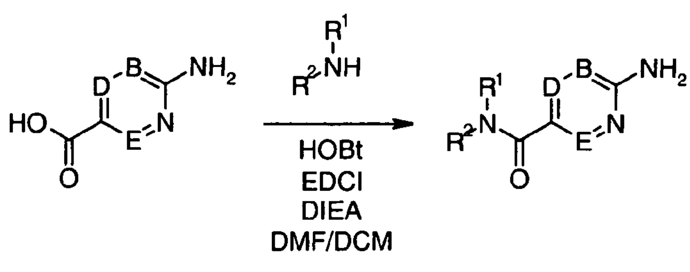


As shown in Reaction Scheme 1, optionally substituted amine and 2-amino-pyridine-5-carboxylic acid, 2-amino-pyrimidine-5-carboxylic acid, 5-amino-pyrazine-2-carboxylic acid or 6-aminopyridazine-3-carboxylic acid are reacted in an amide coupling reaction in the presence of a coupling reagent such as EDCI and HOBt in an organic solvent such as a mixture of DMF and DCM at a suitable temperature to yield the corresponding amide.

## Reaction Scheme 2:

## Synthesis of α-bromoketones

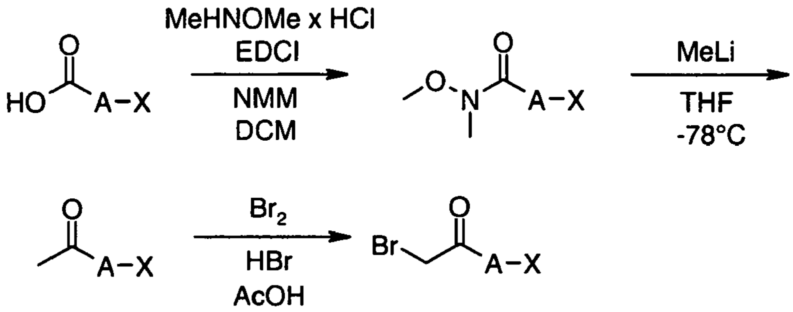


As shown in Reaction Scheme 2, optionally substituted bromoketones can be obtained in a three step reaction sequence starting from carboxylic acids. Said carboxylic acids can be converted to the corresponding Weinreb amides using N,O-dimethylhydroxylamine hydrochloride with a coupling reagent like EDCI in the presence of a suitable base like NMM in an appropriate solvent such as DCM. The Weinreb amides can be converted to the corresponding methyl ketones using a reagent such as methyllithium in an inert solvent like THF at a suitable temperature. Bromination can be achieved using a mixture of bromine and hydrogen bromide in acetic acid.

## Reaction Scheme 3:

## Synthesis of imidazo[1,2-a]pyridines, -pyrimidines, -pyrazines and imidazo[1,2-b]pyridazines

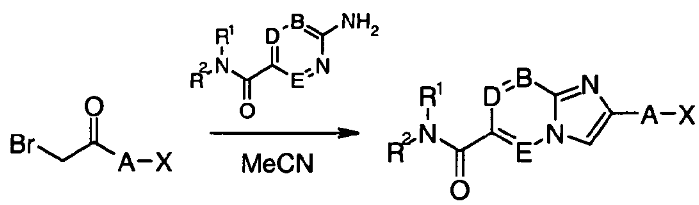


As shown in Reaction Scheme 3, optionally substituted aminopyridine-, aminopyrimidine-, aminopyrazine- and aminopyridazine-amides, which can be obtained as shown in Reaction Scheme 1, can be converted to imidazo[1,2-a] pyridine, -pyrimidine and - pyrazine-6-carboxylic acid amides and imidazo[1,2-b]pyridazine-6-carboxylic acid amides by reaction with α-bromoketones in a solvent like MeCN. This reaction can be carried out either in a flask in refluxing solvent or any other appropriate temperature or in a microwave reaction system. The reaction products can be purified by standard procedures or may precipitate directly from the solution upon cooling and may thus be used in subsequent reactions without further purification.

## Reaction Scheme 4:

## Iodination of imidazo[1,2-a]pyridines, -pyrimidines, -pyrazines and imidazo[1,2-b]pyridazines substituted in 2-position

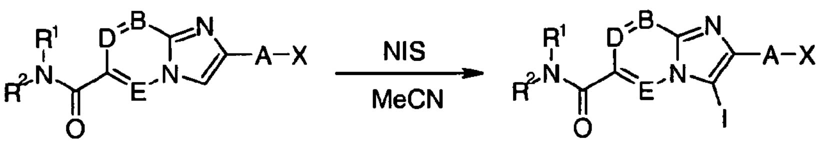

As shown in Reaction Scheme 4 optionally substituted imidazo[1,2-a]pyridine, -pyrimidine and -pyrazine-6-carboxylic acid amides and imidazo[1,2-b]pyridazine-6-carboxylic acid amides can be iodinated in 3-position by treating the starting material with a reagent such as NIS in a suitable solvent like acetonitrile.

**Reaction Scheme 5:**

**Preparation of propargylamines**

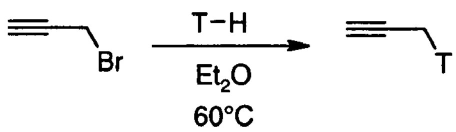


Propargylamines can be prepared as depicted in Reaction Scheme 5. Propargylbromide is reacted with an optionally substituted amine T-H in a solvent like diethyl ether at elevated temperature to yield the desired product.

**Reaction Scheme 6:**

**Sonogashira reaction of 3-iodo-imidazo[1,2-a]pyridines, -pyrimidines, -pyrazines and 3-iodo-imidazo[1,2-b]pyridazines substituted in 2-position**

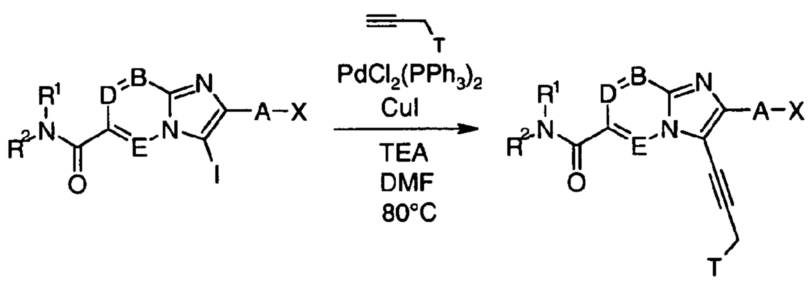


As shown in Reaction Scheme 6 optionally substituted 3-iodo-imidazo[1,2-a]pyridine, -pyrimidine and -pyrazine-6-carboxylic acid amides and 3-iodo-imidazo[1,2-b]pyridazine-6-carboxylic acid amides can be reacted with propargylamines in the presence of a catalyst such as bis(triphenylphosphine)palladium(II) dichloride in the presence of a reagent such as copper(I) iodide and a base like TEA in a suitable solvent like DMF at an appropriate temperature to yield the target compounds.

**Reaction Scheme 7:**

**Synthesis of imidazo[1,2-a]pyridines, -pyrimidines, -pyrazines and imidazo[1,2-b]pyridazines unsubstituted in 2-position**

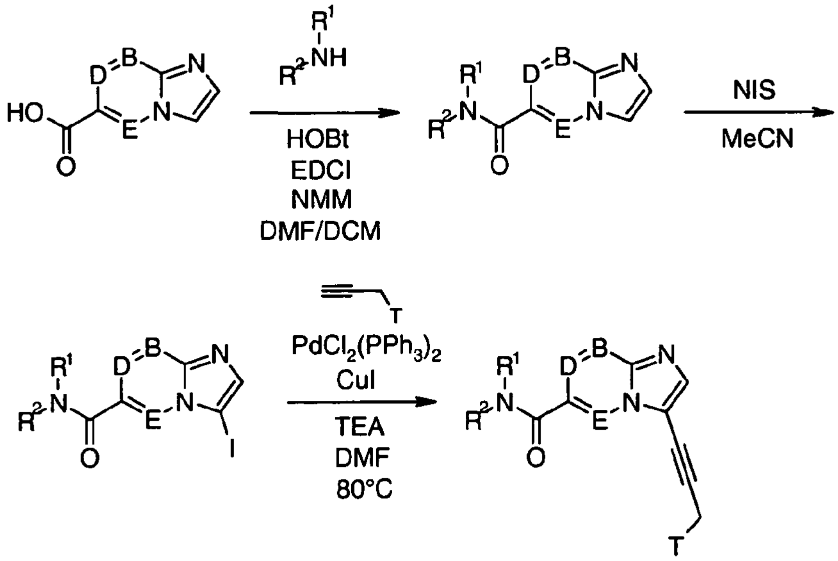


Reaction Scheme 7 shows how imidazo[1,2-a]pyridine, -pyrimidine, -pyrazine-6-carboxylic acid and imidazo [1,2-b]pyridazine-6-carboxylic acid can be reacted with optionally substituted amines in the presence of a coupling reagent such as EDCI and HOBt in a suitable solvent such as a mixture of DMF and DCM. Subsequent treatment with NIS or NBS in a solvent like acetonitrile leads to the corresponding 3-iodo or 3-bromo substituted compounds, respectively. Said compounds can be reacted with propargylamines in the presence of a catalyst such as bis(triphenylphosphine) palladium(II) dichloride and a reagent such as copper(I) iodide and a base like TEA in a suitable solvent like DMF at an appropriate temperature to yield the target compounds.

## Reaction Scheme 8:

## Synthesis of 2-bromo-imidazo[1,2-a]pyridines, -pyrimidines, -pyrazines and 2-bromo-imidazo[1,2-b]pyridazines substituted with a propargylamine moiety in 3-position

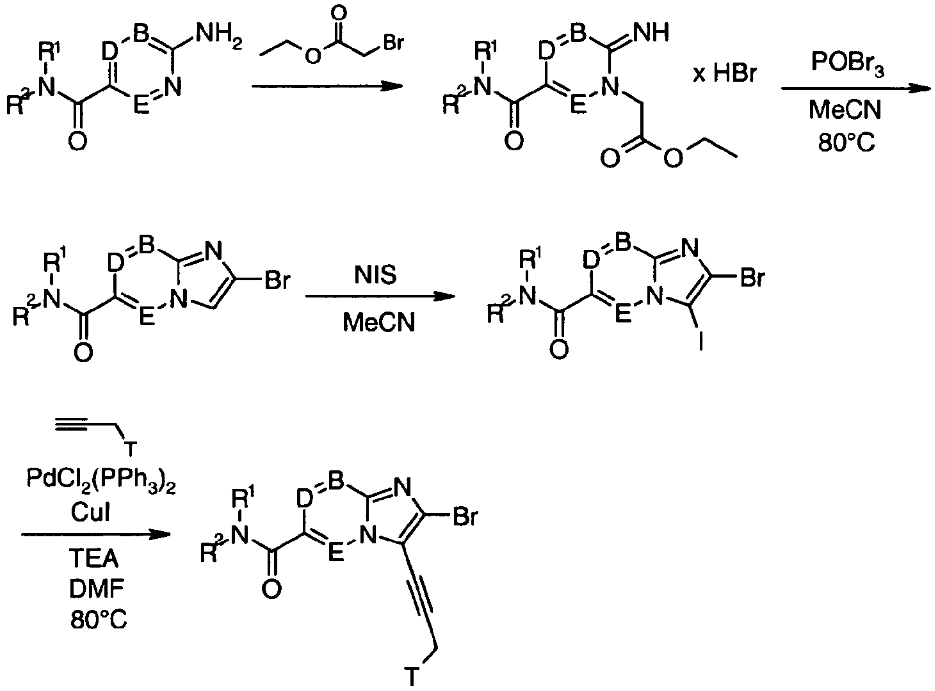


Reaction Scheme 8 shows how optionally substituted 2-amino-pyridine-5-carboxylic acid amides, 2-amino-pyrimidine-5-carboxylic acid amides, 2-amino-pyrazine-5-carboxylic acid amides and 6-amino-pyridazine-3-carboxylic acid amides can be reacted with neat ethyl bromoacetate. The products of this reaction can be cyclized using a reagent such as phosphoryl bromide in a suitable solvent like acetonitrile at an appropriate temperature. Alternatively, the cyclization can also be accomplished in neat phosphoryl chloride at 120˚C to yield 2-chlorosubstituted heterocycles. 2-Bromo-imidazo[1,2-a]pyridines,- pyrimidines, -pyrazines and 2-bromo-imidazo[1,2-b]pyridazines can be iodinated in 3-position using a reagent such as NIS in a suitable solvent like acetonitrile. Reaction with propargylamines, in the presence of a catalyst such as bis(triphenylphosphine)-palladium(II) dichloride, a copper salt like copper(I) iodide and a suitable base like TEA in an appropriate solvent such as DMF at a given temperature leads to the 3-alkinylated products.

## Reaction Scheme 9:

## Boronic ester formation

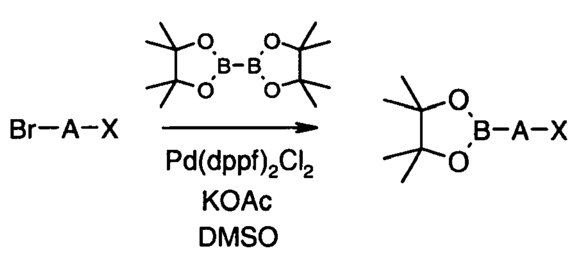


Optionally substituted aryl and heteroarylbromides can be transformed to the corresponding boronic esters as shown in Reaction Scheme 9. Reaction of the bromides with bis(pinacolato)diboron in the presence of a reagent such as potassium acetate and a catalyst like 1,1'-bis(diphenylphosphino)-ferrocene-palladium(II)dichloride dichloromethane

adduct in a solvent such as DMSO at an appropriate temperature yields the target compounds.

**Reaction Scheme 10:**

**Suzuki coupling**

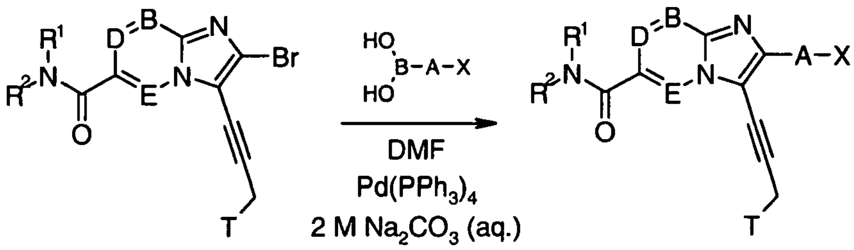


As shown in Reaction Scheme 10, optionally substituted 2-bromo-imidazo[1,2-a]pyridines, -pyrimidines, -pyrazines and 2-bromo-imidazo[1,2-b]pyridazines can be subjected to a Suzuki coupling reaction with boronic acids $(HO)_2B$-A-X or analogues boronic esters using a catalyst such as tetrakis(triphenylphosphine)palladium(0) in the presence of a base such as aqueous sodium carbonate solution in a suitable solvent like DMF at an appropriate temperature to provide the target compounds.

## Reaction Scheme 11:

## Buchwald coupling

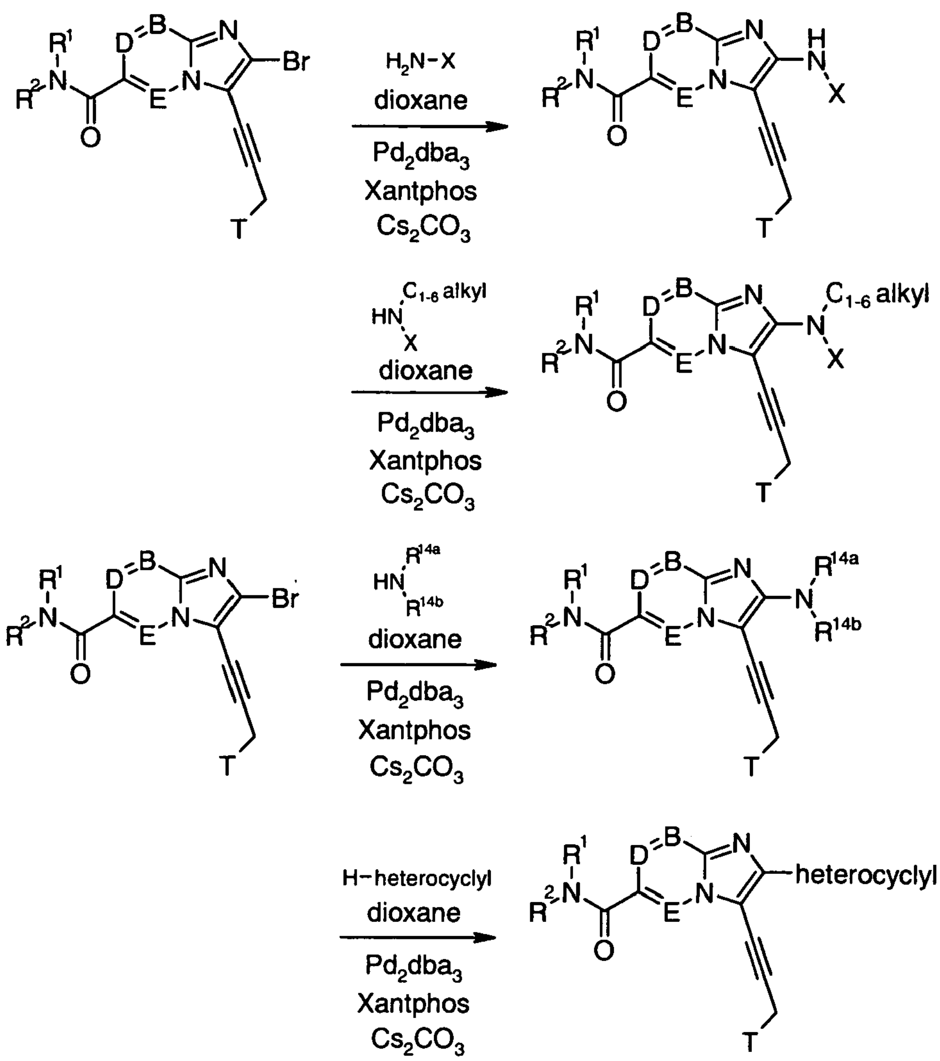


Optionally substituted 2-bromo-imidazo[1,2-a]pyridines, -pyrimidines, -pyrazines and 2-bromo-imidazo[1,2-b] pyridazines can also be reacted with amines under Buchwald conditions, as shown in Reaction Scheme 11. The starting material can be reacted with optionally substituted amines, $H_2N$-X, HN($C_{1-6}$alkyl)-X, $HNR^{14a}R^{14b}$ or H-heterocyclyl (e.g. pyrrolidine, piperidine, morpholine and the like) in the presence of a palladium source like tris(dibenzylideneacetone) dipalladium(0) and a ligand such as 4,5-bis(diphenylphosphino) -9,9-dimethylxanthene in an appropriate solvent like dioxane in the presence of a suitable base such as cesium carbonate at a given temperature.

**Reaction Scheme 12:**

**Sonogashira coupling**

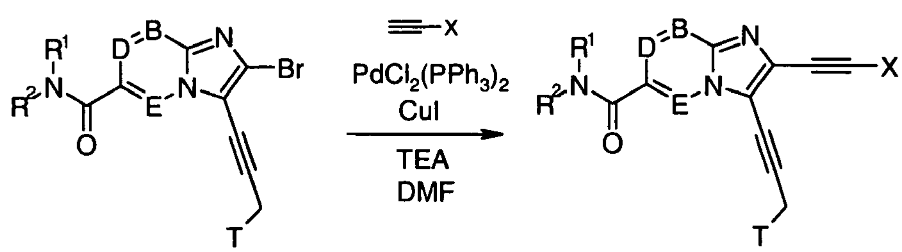


As shown in Reaction Scheme 12, optionally substituted 2-bromo-imidazo[1,2-a]pyridines, -pyrimidines, -pyrazines and 2-bromo-imidazo[1,2-b]pyridazines can be reacted in a Sonogashira coupling with optionally substituted alkynes in the presence of a catalyst such as bis(triphenylphosphine)palladium(II) dichloride, a reagent such as copper (I) iodide and a base like TEA in a suitable solvent like DMF at an appropriate temperature to yield the target compounds.

**Reaction Scheme 13:**

**Heck coupling**

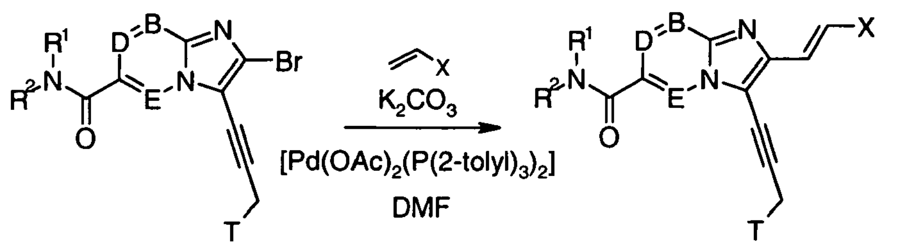


The intermediates from Reaction Scheme 8, optionally substituted 2-bromo-imidazo[1,2-a]pyridines, -pyrimidines, -pyrazines and 2-bromo-imidazo[1,2-b]pyridazines can alternatively be subjected to a Heck coupling. Reaction with an optionally substituted alkene in the presence of a catalyst like $[Pd(OAc)_2(P(2\text{-tolyl})_3)_2]$ (Herrmann, W.A. et al., Angew. Chem. 1995, 107, 1989-1992) and a base such as potassium carbonate in a suitable solvent like DMF at an appropriate temperature yields the target compounds.

**Reaction Scheme 14:**

**Reaction with $Zn(CN)_2$**

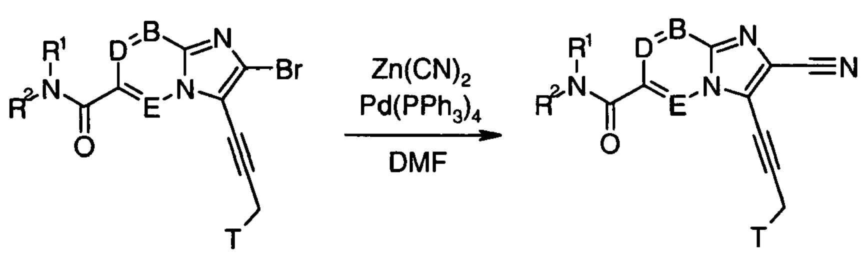


As shown in Reaction Scheme 14, optionally substituted 2-bromo-imidazo[1,2-a]pyridines, -pyrimidines, -pyrazines and 2-bromo-imidazo[1,2-b]pyridazines can also be reacted with a reagent like zinc cyanide in the presence of a catalyst such as tetrakis-(triphenylphosphine)palladium(0) in a suitable solvent like DMF at an appropriate temperature to yield the corresponding nitriles.

## Reaction Scheme 15:

## Stille coupling

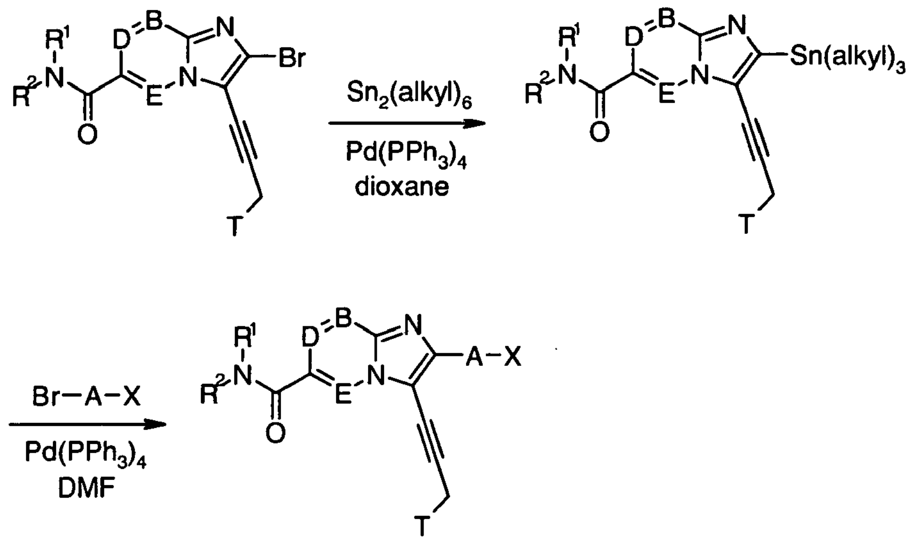


As shown in Reaction Scheme 15, optionally substituted 2-bromo-imidazo[1,2-a]pyridines, -pyrimidines, -pyrazines and 2-bromo-imidazo[1,2-b]pyridazines can be reacted with a reagent like hexaalkylditin in the presence of a catalyst such as tetrakis-(triphenylphosphine)palladium(0) in a suitable solvent like 1,4-dioxane at an appropriate temperature to yield the corresponding 2-trialkylstannyl-imidazo[1,2-a]pyridines, -pyrimidines, -pyrazines and 2-trialkylstannyl-imidazo[1,2-b]pyridazines. Subsequent reaction of these products with optionally substituted arylhalides or heteroarylhalides in the presence of a catalyst such as tetrakis-(triphenylphosphine)palladium(0) in a solvent like DMF at a given temperature leads to the target compounds.

**Analytical LC-MS**

The compounds of the present invention according to formula (I) were analyzed via analytical LC-MS. The conditions used in the analysis are summarized below.

Analytical conditions summary:

LC10Advp-Pump (Shimadzu) with SPD-M10Avp UV/Vis diode array detector and QP2010 MS-detector in ESI+ modus with UV-detection at 220 and 254 nm,
Column: Waters XTerra MS C18, 3.5 μm, 2.1 * 100 mm,
linear gradient with acetonitrile in water (0.15% HCOOH)
Flow rate of 0,4 ml/min;

| | |
|---|---|
| Mobile Phase A: | water (0.15% HCOOH + 5% acetonitrile) |
| Mobile Phase B: | acetonitrile (0.15% HCOOH + 5% water) |

**Gradient A:**

start concentration 15% acetonitrile (0.15% HCOOH)

| | | |
|---|---|---|
| 12.00 | B.Conc | 99 |
| 15.00 | B.Conc | 99 |
| 15.20 | B.Conc | 15 |
| 18.00 | Pump | STOP |

**Gradient B:**

start concentration 10% acetonitrile (0.15% HCOOH)

| | | |
|---|---|---|
| 10.00 min | B.Conc | 60 |
| 11.00 min | B.Curve | 2 |
| 12.00 min | B.Conc | 99 |
| 15.00 min | B.Conc | 99 |
| 15.20 min | B.Conc | 10 |
| 18.00 min | Pump | STOP |

The following describes the detailed examples of the invention which can be prepared via the reaction schemes 1 to 15.

**Table 1:**

| 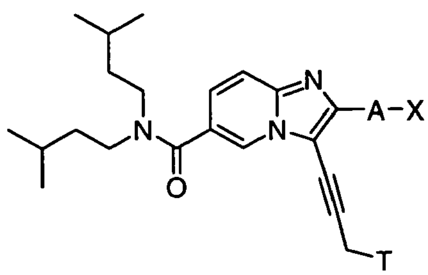 | | | | | | | |
|---|---|---|---|---|---|---|---|
| | | | | HPLC | | MS | |
| No. | salt | A-X | T | $t_R$ (min) | method | MW (calc.) free base | $[M+H]^+$ (found) |
| 1 | - | 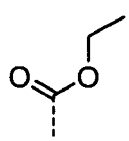 | 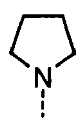 | 6.45 | B | 480.65 | 482 |
| 2 | HCOOH | 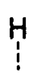 | 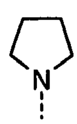 | 5.02 | B | 408.59 | 409 |
| 3 | HCOOH | 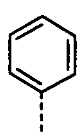 | 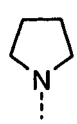 | 5.40 | A | 484.68 | 485 |

(continued)

| | | | | HPLC | | MS | |
|---|---|---|---|---|---|---|---|
| No. | salt | A-X | T | $t_R$ (min) | method | MW (calc.) free base | $[M+H]^+$ (found) |
| 4 | HCOOH | 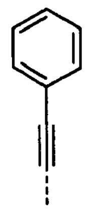 | 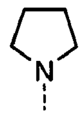 | 8.35 | B | 508.71 | 509 |
| 5 | - | 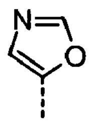 | 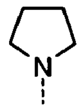 | 6.55 | B | 475.63 | 477 |
| 6 | - | 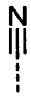 | 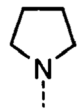 | 6.67 | B | 433.60 | 435 |
| 7 | HCOOH | 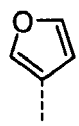 | 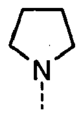 | 7.30 | B | 474.65 | 475 |
| 8 | HCOOH | 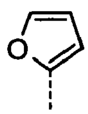 | 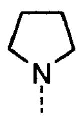 | 7.39 | B | 474.65 | 475 |
| 9 | - | 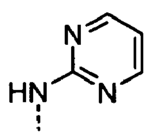 | 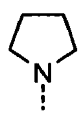 | 5.35 | B | 501.68 | 503 |
| 10 | - | 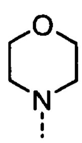 | 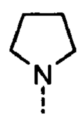 | 6.26 | B | 493.69 | 494 |
| 11 | - | 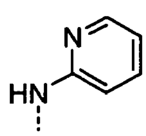 | 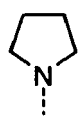 | 4.89 | B | 500.69 | 501 |
| 12 | - | 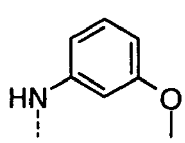 | 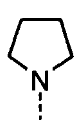 | 7.68 | B | 529.72 | 530 |

(continued)

| | | | | HPLC | | MS | |
|---|---|---|---|---|---|---|---|
| No. | salt | A-X | T | $t_R$ (min) | method | MW (calc.) free base | $[M+H]^+$ (found) |
| 13 | - | 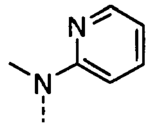 | 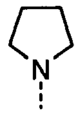 | 5.46 | B | 514.71 | 515 |
| 14 | - | 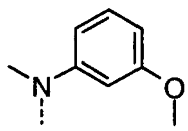 | 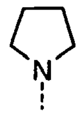 | 7.81 | B | 543.75 | 544 |
| 15 | - | 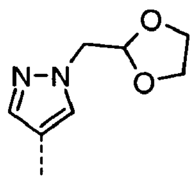 | 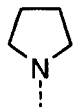 | 6.22 | B | 560.74 | 561 |
| 16 | - | 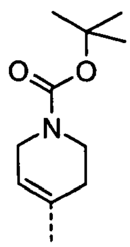 | 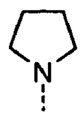 | 8.13 | B | 589.82 | 591 |
| 17 | - | 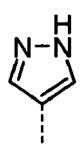 | 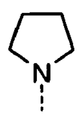 | 5.91 | B | 474.65 | 475 |
| 18 | - | 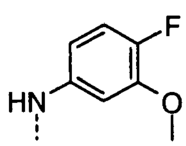 | 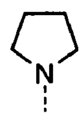 | 8.46 | B | 547.72 | 548 |
| 19 | - | 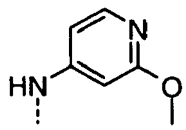 | 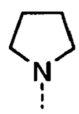 | 5.22 | B | 530.72 | 531 |
| 20 | - | 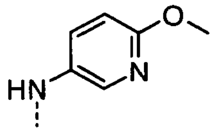 | 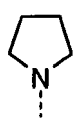 | 7.09 | B | 530.72 | 531 |

(continued)

| | | | | HPLC | | MS | |
|---|---|---|---|---|---|---|---|
| No. | salt | A-X | T | $t_R$ (min) | method | MW (calc.) free base | $[M+H]^+$ (found) |
| 21 | - | 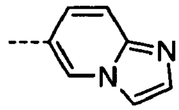 | 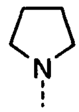 | 4.66 | B | 524.71 | 525 |
| 22 | - | 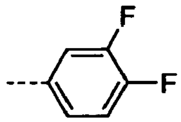 | 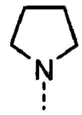 | 8.37 | B | 520.66 | 521 |
| 23 | - | 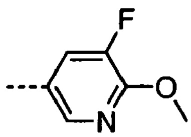 | 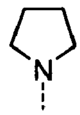 | 7.71 | B | 533.69 | 534 |
| 24 | - | 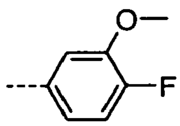 | 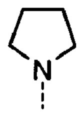 | 8.09 | B | 532.70 | 533 |
| 25 | - | 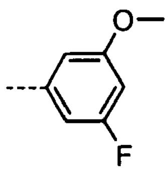 | 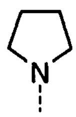 | 8.16 | B | 532.70 | 533 |
| 26 | - | 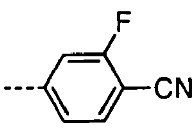 | 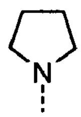 | 8.02 | B | 527.68 | 528 |
| 27 | - | 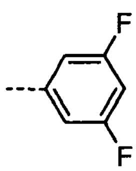 | 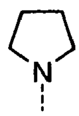 | 8.09 | B | 520.66 | 521 |
| 28 | - | 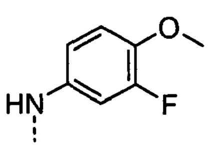 | 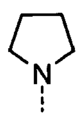 | 8.38 | B | 547.72 | 548 |
| 29 | - | 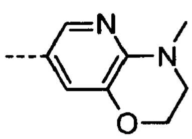 | 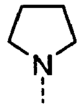 | 6.06 | B | 556.75 | 557 |

(continued)

| | | | | HPLC | | MS | |
|---|---|---|---|---|---|---|---|
| No. | salt | A-X | T | $t_R$ (min) | method | MW (calc.) free base | $[M+H]^+$ (found) |
| 30 | - | 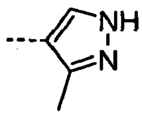 | 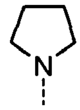 | 6.23 | B | 488.68 | 489 |
| 31 | - | 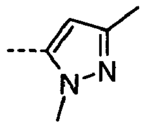 | 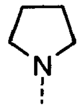 | 6.78 | B | 502.70 | 503 |
| 32 | - | 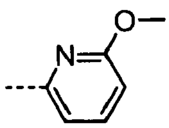 | 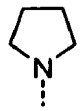 | 7.60 | B | 515.70 | 516 |
| 33 | HCOOH | 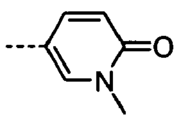 | 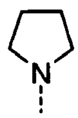 | 6.10 | B | 515.70 | 516 |
| 34 | - | 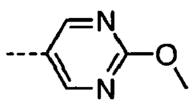 | 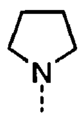 | 6.59 | B | 516.69 | 517 |
| 35 | HCOOH | 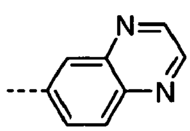 | 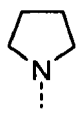 | 7.12 | B | 536.72 | 537 |
| 36 | - | 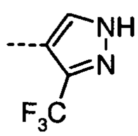 | 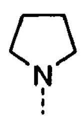 | 7.30 | B | 542.65 | 543 |

The following examples are provided to illustrate the invention and are not limiting the scope of the invention in any manner.

**Common Intermediates:**

***6-Amino-N,N-bis-(3-methyl-butyl)-nicotinamide:***

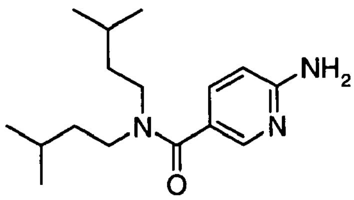

6-Aminonicotinic acid (20.0 g) was dissolved in DMF (300 ml) and DCM (75 ml) and treated with diisopentylamine (36.0 ml), EDCI (34.0 g), HOBt (26.0 g) and N,N-diisopropylethylamine (30.0 ml) in this order, stirred at 50˚C overnight, and completely evaporated. The residue was re-dissolved in ethyl acetate and washed with brine, saturated sodium bicarbonate solution and brine. The organic layer was dried over sodium sulfate, filtered, and evaporated. The crude product was purified by column chromatography.

***{5-[Bis-(3-methyl-butyl)-carbamoyl]-2-imino-1,2-dihydropyridin-1-yl}-acetic acid ethyl ester hydrobromide:***

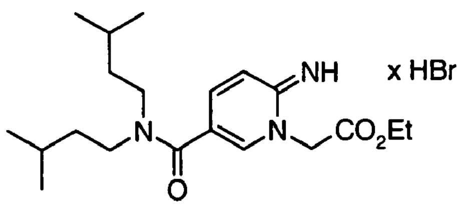

A solution of 6-amino-N,N-bis-(3-methyl-butyl)-nicotinamide (10.0 g) in ethyl bromoacetate (25 ml) was stirred at 22˚C for 16 h. The reaction mixture was carefully evaporated under high vaccum (0.1 mbar/50˚C), co-evaporated with toluene (5 x 100 ml), and dried under high vaccum to give the crude product as brown solid, which was used in the next step without any further purification.

***2-Bromo-imidazo[1,2-a]pyridine-6-carboxylic acid bis-(3-methyl-butyl)amide:***

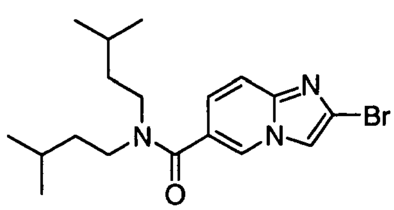

At 22˚C, a solution of phosphorus(V) oxybromide (20.7 g) in acetonitrile (40 ml) was added dropwise over a time period of 20 min to a solution of the crude {5-[bis-(3-methylbutyl)-carbamoyl]-2-imino-1,2-dihydropyridin-1-yl}-acetic acid ethyl ester hydrobromide (17.1 g) in acetonitrile (110 ml). The reaction mixture was transferred to a pre-heated oil bath (80˚C), and stirred 18 h at this temperature. The reaction mixture was cooled to 22˚C, diluted with ethyl acetate (350 ml) and slowly poured onto a mixture of ice and saturated sodium bicarbonate solution (300 ml). After separation, the organic layer was washed with saturated sodium bicarbonate solution (3 x 200 ml). The combined aqueous layer was extracted with ethyl acetate (4 x 150 ml). The combined organic extract was washed with brine (400 ml), dried over $Na_2SO_4$, filtered, and evaporated. The crude material was purified by flash chromatography.

***2-Bromo-3-iodo-imidazo[1,2-a]pyridine-6-carboxylic acid bis-(3-methyl-butyl)amide:***

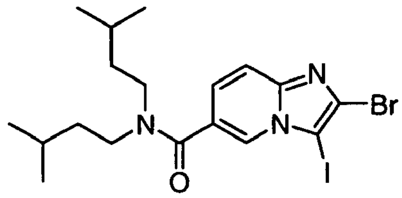

A solution of 2-bromo-imidazo[1,2-a]pyridine-6-carboxylic acid bis-(3-methyl-butyl)amide (4.47 g) in acetonitrile (90 ml) was treated with N-iodosuccinimide (2.90 g) and stirred in the absence of light at 22˚C for 1 h. The reaction mixture was diluted with diethyl ether (150 ml) and washed with 1 M aqueous sodium thiosulfate solution (3 x 100 ml). The combined aqueous layer was washed with diethyl ether (2 x 80 ml). The combined organic extract was washed with water (100 ml) and brine (100 ml), dried over sodium sulfate, filtered, and evaporated to give the crude product, which was used in the next step without any further purification.

***2-Bromo-3-(3-pyrrolidin-1-yl-prop-1-ynyl)-imidazo[1,2-a]pyridine-6-carboxylic acid bis-(3-methyl-butyl)amide:***

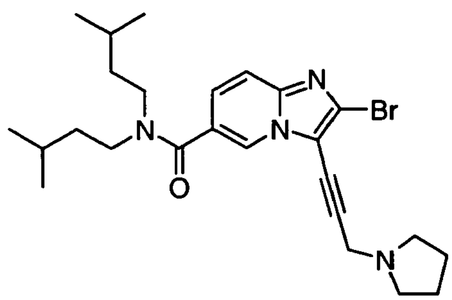

A degassed mixture of 2-bromo-3-iodo-imidazo[1,2-a]pyridine-6-carboxylic acid bis-(3-methyl-butyl)amide (6.01 g), $Pd(PPh_3)_2Cl_2$ (412 mg), copper(I) iodide (336 mg), and triethylamine (8.2 ml) in DMF (185 ml) was treated with N-propargyl pyrrolidine (Biel and DiPierro, J. Am. Chem. Soc. 1958, 80, 4609-4614) (2.9 ml) and stirred at 80˚C for 1 h. The reaction mixture was diluted with diethyl ether (150 ml) and washed with water (3 x 200 ml). The combined aqueous layer was extracted with diethyl ether (4 x 150 ml). The combined organic extract was washed with brine (250 ml), dried over sodium sulfate, filtered, and evaporated. The crude product was purified by flash chromatography.

**Synthesis of Example 1:**

***Intermediate 1a):***

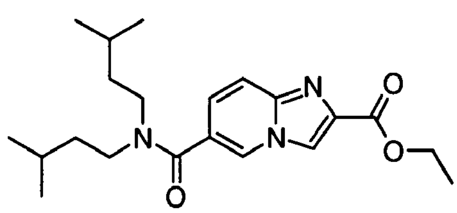

6-Amino-N,N-bis-(3-methyl-butyl)-nicotinamide (971 mg) was added to ethyl bromopyruvate (939 mg) in ethanol (10 ml) and the reaction mixture was heated to 120˚C using microwave irradiation for 40 min. Volatiles were removed and the residue was purified by column chromatography.

***Intermediate 1b):***

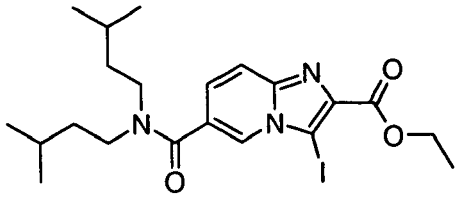

A solution of 6-[bis-(3-methyl-butyl)-carbamoyl]-imidazo[1,2-a]pyridine-2-carboxylic acid ethyl ester (180 mg) in acetonitrile (5 ml) was treated with N-iodosuccinimide (120 mg) and stirred in the absence of light at 22°C for 2 h. The reaction mixture was diluted with diethyl ether (30 ml) and washed with 1 M aqueous sodium thiosulfate solution (3 x 25 ml). The combined aqueous layer was washed with diethyl ether (1 x 20 ml). The combined organic extract was washed with water (35 ml) and brine (35 ml), dried over sodium sulfate, filtered, and evaporated. The crude product was purified by column chromatography.

***Example 1:***

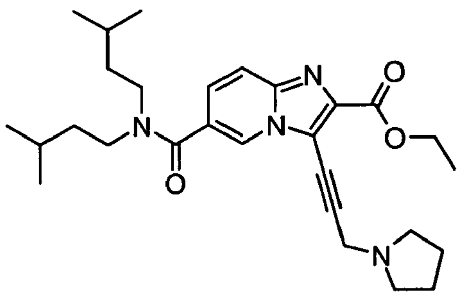

A degassed mixture of 6-[bis-(3-methyl-butyl)-carbamoyl]-3-iodo-imidazo[1,2-a]pyridine-2-carboxylic acid ethyl ester (61 mg), $Pd(PPh_3)_2Cl_2$ (4.3 mg), copper(I) iodide (3.5 mg), and triethylamine (86 μl) in DMF (2 ml) was treated with N-propargyl pyrrolidine (61 μl) and stirred at 80°C for 2 h. The reaction mixture was diluted with diethyl ether (25 ml) and washed with water (3 x 20 ml). The combined aqueous layer was washed with diethyl ether (2 x 20 ml). The combined organic extract was washed with brine (40 ml), dried over sodium sulfate, filtered, and evaporated. The desired product was isolated by column chromatography and re-purified by preparative HPLC-MS.

**Synthesis of Example 2:**

***Intermediate 2a):***

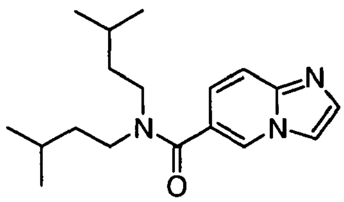

A suspension of imidazo[1,2-a]pyridine-6-carboxylic acid (1.0 g), EDCI (1.775 g), HOBt (1.415 g) and N-methylmorpholine (1.0 ml) in DMF (20 ml) and DCM (5 ml) was stirred at 22°C for 40 min. The resulting reddish solution was treated with diisopentylamine (1.9 ml) and N-methylmorpholine (1.0 ml) in this order and the reaction mixture was stirred at 22°C for 24 h. The mixture was diluted with diethyl ether (100 ml) and washed with saturated sodium bicarbonate solution (2 x 60 ml). The combined aqueous layer was washed with diethyl ether (2 x 50 ml). The combined organic extract was washed with water (100 ml) and brine (100 ml), dried over sodium sulfate, filtered, and evaporated. The crude product was purified by column chromatography.

***Intermediate 2b):***

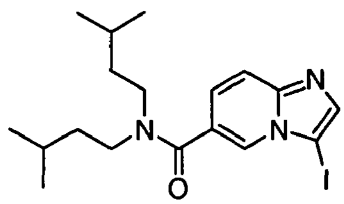

A solution of imidazo[1,2-a]pyridine-6-carboxylic acid bis-(3-methyl-butyl)amide (150 mg) in acetonitrile (1.8 ml) was treated with N-iodosuccinimide (125 mg) and stirred in the absence of light at 22°C for 90 min. The reaction mixture was diluted with diethyl ether (25 ml) and washed with 1M aqueous sodium thiosulfate solution (3 x 20 ml). The combined aqueous layer was washed with diethyl ether (2 x 20 ml). The combined organic extract was washed with water (40 ml) and brine (40 ml), dried over sodium sulfate, filtered, and evaporated. The crude product was purified by column chromatography.

***Example 2:***

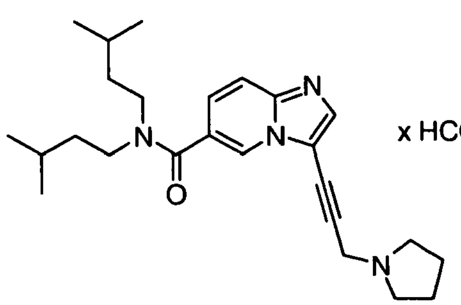

x HCOOH

A degassed mixture of 3-iodo-imidazo[1,2-a]pyridine-6-carboxylic acid bis-(3-methylbutyl)amide (320 mg), Pd $(PPh_3)_2Cl_2$ (25 mg), copper(I) iodide (21 mg), and triethylamine (0.52 ml) in DMF (14 ml) was treated with N-propargyl

pyrrolidine (0.37 ml) and stirred at 80°C for 2.5 h. The reaction mixture was diluted with diethyl ether (40 ml) and washed with water (3 x 30 ml). The combined aqueous layer was washed with diethyl ether (2 x 30 ml). The combined organic extract was washed with brine (50 ml), dried over sodium sulfate, filtered, and evaporated. The desired product was isolated by column chromatography and re-purified by preparative HPLC-MS.

**Synthesis of Example 4:**

***Example 4:***

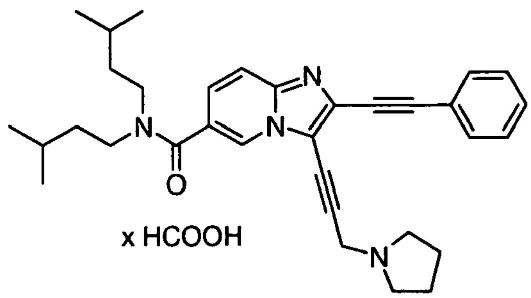

x HCOOH

A degassed mixture of 2-bromo-3-(3-pyrrolidin-1-yl-prop-1-ynyl)-imidazo[1,2-a]pyridine-6-carboxylic acid bis-(3-methyl-butyl)amide (50 mg), $Pd(PPh_3)_2Cl_2$ (3.6 mg), copper(I) iodide (2.9 mg), and triethylamine (72 μl) in DMF (2 ml) was treated with phenyl acetylene (47 μl) and stirred at 90°C for 2.5 h and at 110°C for additional 5.5 h. The reaction mixture was diluted with diethyl ether (30 ml) and washed with saturated aqueous sodium bicarbonate solution (3 x 25 ml). The combined aqueous layer was extracted with diethyl ether (2 x 20 ml). The combined organic extract was washed with water (40 ml) and brine (40 ml), dried over sodium sulfate, filtered, and evaporated. The crude product was purified by preparative HPLC-MS to provide the title compound as a mono-formate salt.

**Synthesis of Example 6:**

***Example 6:***

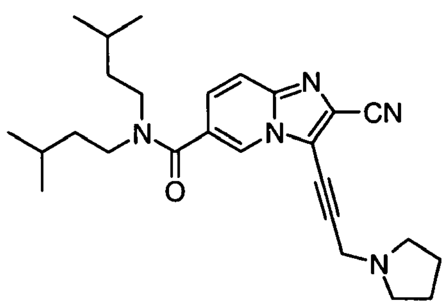

A degassed mixture of 2-bromo-3-(3-pyrrolidin-1-yl-prop-1-ynyl)-imidazo[1,2-a]pyridine-6-carboxylic acid bis-(3-methyl-butyl)amide (100 mg) and zinc cyanide (48 mg) was dissolved in DMF (1 ml) at 22°C and treated with $Pd(PPh_3)_4$ (71 mg). The reaction mixture was transferred to a pre-heated oil bath (130°C) and stirred in a sealed tube at this temperature for 2 h. The reaction mixture was diluted with diethyl ether (25 ml) and washed with 1 M aqueous ammonium chloride solution (3 x 25 ml). The combined aqueous layer was extracted with diethyl ether (2 x 20 ml). The combined organic extract was washed with water (30 ml) and brine (30 ml), dried over sodium sulfate, filtered, and evaporated. The crude product was purified by column chromatography.

**Synthesis of Example 10:**

***Example 10:***

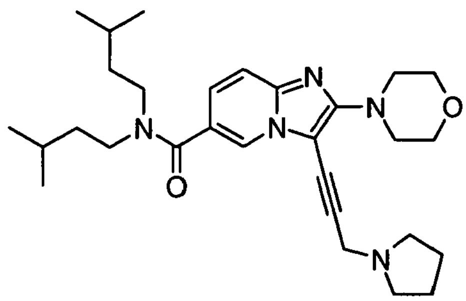

At 22˚C, a mixture of 2-bromo-3-(3-pyrrolidin-1-yl-prop-1-ynyl)-imidazo[1,2-a]pyridine-6-carboxylic acid bis-(3-methyl-butyl)amide (50 mg) and morpholine (18 μl) in 1,4-dioxane (1 ml) was subjected to 3 cycles of evacuation/backfilling with Ar procedure and then treated successively with $Pd_2dba_3$ (9.4 mg) and 4,5-bis(diphenylphosphino)-9,9-dimethylxanthene (17.8 mg). The evacuation/backfilling with Ar procedure was repeated three times and cesium carbonate (67 mg, freshly dried with a heat gun under high vacuum before use) was added in one portion. The evacuation/backfilling with Ar procedure was repeated three times. The reaction mixture was transferred to a pre-heated oil bath (110˚C) and stirred in a sealed tube at this temperature for 5 h. The mixture was diluted with ethyl acetate (25 ml) and washed with water (2 x 20 ml). The combined aqueous layer was extracted with ethyl acetate (2 x 20 ml). The combined organic extract was washed with brine (35 ml), dried over sodium sulfate, filtered, and evaporated. The crude product was purified by column chromatography.

**Synthesis of Example 11:**

***Example 11:***

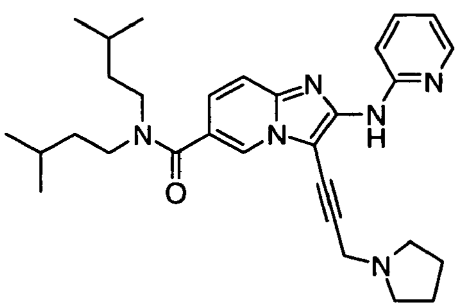

At 22˚C, a mixture of 2-bromo-3-(3-pyrrolidin-1-yl-prop-1-ynyl)-imidazo[1,2-a]pyridine-6-carboxylic acid bis-(3-methyl-butyl)amide (62 mg) and 2-aminopyridine (27 mg) in 1,4-dioxane (1.5 ml) was subjected to 3 cycles of evacuation/backfilling with Ar procedure and then treated successively with $Pd_2dba_3$ (13.2 mg) and 4,5-bis(diphenylphosphino)-9,9-dimethylxanthene (25 mg). The evacuation/backfilling with Ar procedure was repeated three times and cesium carbonate (94 mg, freshly dried with a heat gun under high vacuum before use) was added in one portion. The evacuation/backfilling with Ar procedure was repeated three times. The reaction mixture was transferred to a pre-heated oil bath (110˚C) and stirred in a sealed tube at this temperature for 5 h. The mixture was diluted with ethyl acetate (25 ml) and

washed with water (3 x 25 ml). The combined aqueous layer was extracted with ethyl acetate (1 x 25 ml). The combined organic extract was washed with brine (40 ml), dried over sodium sulfate, filtered, and evaporated. The crude product was purified by column chromatography.

**Synthesis of Example 13:**

***Example 13:***

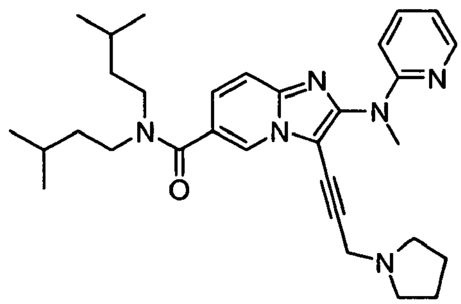

At 22°C, a mixture of 2-bromo-3-(3-pyrrolidin-1-yl-prop-1-ynyl)-imidazo[1,2-a]pyridine-6-carboxylic acid bis-(3-methyl-butyl)amide (50 mg) and 2-(methylamino)pyridine (21 μl) in 1,4-dioxane (1 ml) was subjected to 3 cycles of evacuation/backfilling with Ar procedure and then treated successively with $Pd_2dba_3$ (9.4 mg) and 4,5-bis(diphenylphosphino)-9,9-dimethylxanthene (17.8 mg). The evacuation/backfilling with Ar procedure was repeated three times and cesium carbonate (67 mg, freshly dried with a heat gun under high vacuum before use) was added in one portion. The evacuation/backfilling with Ar procedure was repeated three times. The reaction mixture was transferred to a pre-heated oil bath (110°C) and stirred in a sealed tube at this temperature for 4.5 h. The mixture was diluted with ethyl acetate (20 ml) and washed with water (1 x 25 ml). The aqueous layer was extracted with ethyl acetate (1 x 15 ml). The combined organic extracts were washed with brine (30 ml), dried over sodium sulfate, filtered, and evaporated. The crude product was purified by column chromatography.

**Synthesis of Example 17:**

***Example 17:***

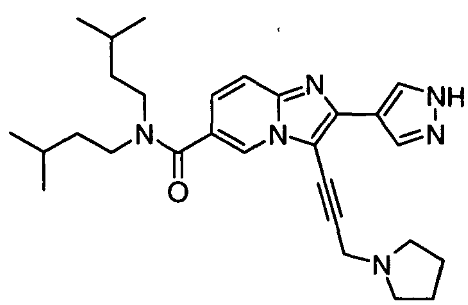

A degassed mixture of 2-bromo-3-(3-pyrrolidin-1-yl-prop-1-ynyl)-imidazo[1,2-a]pyridine-6-carboxylic acid bis-(3-methyl-butyl)amide (50 mg), 4-pyrazoleboronic acid pinacol ester (30 mg), and 2M aqueous sodium carbonate solution (0.2 ml) in DMF (1 ml) was treated with $Pd(PPh_3)_4$ (12.0 mg). The reaction mixture was transferred to a pre-heated oil bath (110°C) and stirred in a sealed tube at this temperature for 3 h. The mixture was diluted with diethyl ether

(25 ml) and washed with water (1 x 20 ml). The aqueous layer was extracted with diethyl ether (2 x 15 ml). The combined organic extract was washed with brine (30 ml), dried over sodium sulfate, filtered, and evaporated. The crude product was purified by column chromatography.

**Synthesis of Example 33:**

***Intermediate 33a:***

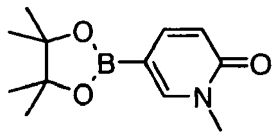

To a solution of 5-bromo-1-methylpyridin-2(1H)-one (800 mg) in DMSO (21 ml) was added bis(pinacolato) diboron (1620 mg), potassium acetate (1253 mg) and 1,1'-bis(diphenyl-phosphino)ferrocene-palladium(II)dichloride dichloromethane adduct (213 mg). The reaction mixture was stirred at 80˚C for 30 min. The reaction was diluted with water (20 ml) and extracted with EtOAc (3 x 30 ml). The organic layer was dried with $Na_2SO_4$ and solvents were reduced under reduced pressure. The crude product was purified by flash chromatography.

***Example 33:***

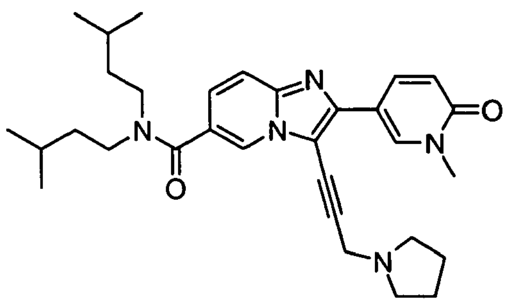

A degassed mixture of 2-bromo-3-(3-pyrrolidin-1-yl-prop-1-ynyl)-imidazo[1,2-a]pyridine-6-carboxylic acid bis-(3-methyl-butyl)amide (200 mg), intermediate 33a) (145 mg), and 2M aqueous sodium carbonate solution (0.8 ml) in DMF (4 ml) was treated with $Pd(PPh_3)_4$ (47.3 mg). The reaction mixture was transferred to a pre-heated oil bath (110˚C) and stirred in a sealed tube at this temperature overnight. The mixture was diluted with diethyl ether (50 ml) and washed with water (1 x 20 ml). The aqueous layer was extracted with diethyl ether (2 x 20 ml). The combined organic extract was washed with brine (50 ml), dried over sodium sulfate, filtered, and evaporated. The crude product was purified by column chromatography followed by preparative LC-MS.

**Synthesis of Example 35:**

***Intermediate 35a:***

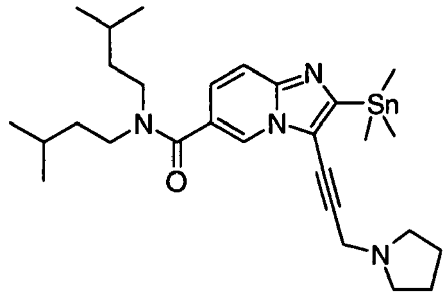

A degassed mixture of 2-bromo-3-(3-pyrrolidin-1-yl-prop-1-ynyl)-imidazo[1,2-a]pyridine-6-carboxylic acid bis-(3-methyl-butyl)amide (150 mg) and hexamethylditin (303 mg) in 1,4-dioxane (3 ml) was treated with $Pd(PPh_3)_4$ (36 mg). The reaction mixture was transferred to a pre-heated oil bath (110˚C) and stirred in a sealed tube at this temperature for 90 min. The mixture was cooled down to 23˚C, treated with 1 M aqueous KF solution (10 ml) and extracted with ethyl acetate (2 x 10 ml). The combined organic extracts were washed with brine (10 ml), dried over sodium sulfate, filtered, and evaporated. The crude product was used in the next step without any further purification.

***Example 35:***

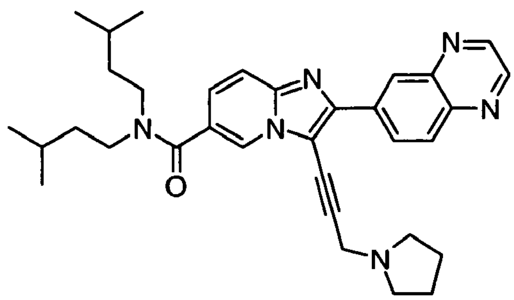

A degassed mixture of the crude 3-(3-pyrrolidin-1-yl-prop-1-ynyl)-2-trimethylstannanyl-imidazo[1,2-a]pyridine-6-carboxylic acid bis-(3-methyl-butyl)amide (ca. 262 mg) and 6-bromoquinoxaline (96 mg) in DMF (3 ml) was treated with $Pd(PPh_3)_4$ (48 mg). The reaction mixture was transferred to a pre-heated oil bath (110˚C) and stirred in a sealed tube at this temperature for 18 h. The mixture was diluted with diethyl ether (25 ml) and washed with water (1 x 20 ml). The aqueous layer was extracted with diethyl ether (2 x 15 ml). The combined organic extracts were washed with brine (30 ml), dried over sodium sulfate, filtered, and evaporated. The crude product was isolated by column chromatography and re-purified by preparative HPLC-MS.

**BIOLOGICAL ASSAYS**

**A. Binding Assay**

A membrane binding assay is used to identify competitive inhibitors of fluorescence labeled NDP-alpha-MSH binding to HEK293 cell membrane preparations expressing human melanocortin receptors.

The test compound or unlabeled NDP-alpha-MSH is dispensed at varying concentrations to a 384 well microtiter plate. Fluorescence labeled NDP-alpha-MSH is dispensed at a single concentration, followed by addition of membrane preparations. The plate is incubated for 5 h at room temperature.

The degree of fluorescence polarization is determined with a fluorescence polarization microplate reader.

**B. Functional Assay**

Agonistic activity of human melanocortin receptors is determined in a homogeneous membrane based assay. Competition between unlabeled cAMP and a fixed quantity of fluorescence labeled cAMP for a limited number of binding sites on a cAMP specific antibody is revealed by fluorescence polarization.

The test compound or unlabeled NDP-alpha-MSH is dispensed at varying concentrations to a 384 well microtiter plate. Membrane preparations from HEK293 cells expressing the human melanocortin receptors are added. After a short preincubation period, an appropriate amount of ATP, GTP and the cAMP antibody is added and the plate is further incubated before the fluorescence labeled cAMP conjugate is dispensed. The plate is incubated for 2 h at 4˚C before it is read on a fluorescence polarization microplate reader. The amount of cAMP produced as a response to a test compound is compared to the production of cAMP resulting from stimulation with NDP-alpha-MSH.

Representative compounds of the present invention were tested and found to bind to the melanocortin-4 receptor. These compounds were generally found to have $IC_{50}$ values less than 2 μM. Representative compounds of the present invention were also tested in the functional assay and found generally not to activate the melanocortin-4 receptor.

**Table 2: Biological data for the examples of the invention**

In the table are listed the $IC_{50}$ values of the hMC-4R binding assay and the $EC_{50}$ values of the functional assay. The $IC_{50}$ and $EC_{50}$ values are grouped in 3 classes:

a ≤ 0.1 μM; b > 0.1 μM and ≤ 1.0 μM; c > 1.0 μM

| Example | hMC-4R binding assay $IC_{50}$/μM | hMC-4R functional assay $EC_{50}$/μM | % activation functional assay |
|---|---|---|---|
| SHU9119 | a | - | 7 |
| NDP-α-MSH | a | a | 100 |
| 1 | b | - | 0 |
| 2 | b | - | 0 |
| 3 | c | - | 0 |
| 4 | b | - | 5 |
| 5 | a | - | 0 |
| 6 | c | - | 6 |
| 7 | b | - | 3 |
| 8 | b | - | 5 |
| 9 | b | - | -12 |
| 10 | b | - | 0 |
| 11 | a | - | -7 |
| 12 | a | - | -3 |
| 13 | b | - | -4 |
| 14 | b | - | 0 |
| 15 | a | - | -4 |
| 16 | c | - | -11 |
| 17 | a | - | -6 |
| 18 | a | - | -5 |
| 19 | a | - | -13 |
| 20 | a | - | -16 |

(continued)

| Example | hMC-4R binding assay $IC_{50}/\mu M$ | hMC-4R functional assay $EC_{50}/\mu M$ | % activation functional assay |
|---|---|---|---|
| 21 | a | - | -12 |
| 22 | b | - | 0 |
| 23 | b | - | -9 |
| 24 | b | - | -2 |
| 25 | c | - | -2 |
| 26 | b | - | -4 |
| 27 | c | - | -3 |
| 28 | a | - | -12 |
| 29 | a | - | -5 |
| 30 | b | - | 4 |
| 31 | b | - | 3 |
| 32 | b | - | -4 |
| 33 | a | | |
| 34 | a | | |
| 35 | a | | |
| 36 | b | | |

## C. In Vivo Food Intake Models

### 1. Spontaneous Feeding Paradigm

Food intake in rats is measured after i.p. or p.o. administration of the test compound (see e.g. Chen, A.S. et al. Transgenic Res 2000 Apr; 9(2):145-54).

### 2. Models of LPS-Induced Anorexia and Tumor-Induced Cachexia

Prevention or amelioration of anorexia induced by lipopolysaccharide (LPS) administration or cachexia induced by tumor growth is determined upon i.p. or p.o. administration of test compounds to rats (see e.g. Marks, D.L.; Ling, N and Cone, R.D. Cancer Res 2001 Feb 15; 61(4):1432-8).

## D. In vitro ADME Assays

### 1. Microsomal Stability

### Experimental Procedure

Pooled human liver microsomes (pooled male and female) and pooled rat liver microsomes (male Sprague Dawley rats) are prepared. Microsomes are stored at -80˚C prior to use.

Microsomes (final concentration 0.5 mg/ml), 0.1 M phosphate buffer pH7.4 and test compound (final substrate concentration = 3 $\mu$M; final DMSO concentration = 0.25%) are pre-incubated at 37˚C prior to the addition of NADPH (final concentration = 1 mM) to initiate the reaction. The final incubation volume is 25 $\mu$l. A control incubation is included for each compound tested where 0.1 M phosphate buffer pH7.4 is added instead of NADPH (minus NADPH). Two control compounds are included with each species. All incubations are performed singularly for each test compound.

Each compound is incubated for 0, 5, 15, 30 and 45 min. The control (minus NADPH) is incubated for 45 min only. The reactions are stopped by the addition of 50 $\mu$l methanol containing internal standard at the appropriate time points. The incubation plates are centrifuged at 2,500 rpm for 20 min at 4˚C to precipitate the protein.

**Quantitative Analysis**

Following protein precipitation, the sample supernatants are combined in cassettes of up to 4 compounds and analysed using generic LC-MS/MS conditions.

**Data Analysis**

From a plot of the peak area ratio (compound peak area/internal standard peak area) against time, the gradient of the line is determined. Subsequently, half-life and intrinsic clearance are calculated using the equations below:

$$\text{Elimination rate constant (k)} = (\text{- gradient})$$

$$\text{Half life } (t_{1/2}) \text{ (min)} = \frac{0.693}{k}$$

$$\text{Intrinsic Clearance } (CL_{int}) \text{ (µl/min/mg protein)} = \frac{V \times 0.693}{t_{1/2}}$$

where V = Incubation volume µl/mg microsomal protein.

Two control compounds are included in the assay and if the values for these compounds are not within the specified limits the results are rejected and the experiment repeated.

**2. Hepatocyte Stability**

**Experimental Procedure**

Suspensions of cryopreserved hepatocytes are used for human hepatocyte stability assay (pooled from 3 individuals). All cryopreserved hepatocytes are purchased from In Vitro Technologies, Xenotech or TCS.
Incubations are performed at a test or control compound concentration of 3 µM at a cell density of $0.5 \times 10^6$ viable cells/mlL. The final DMSO concentration in the incubation is 0.25%. Control incubations are also performed in the absence of cells to reveal any non-enzymatic degradation.
Duplicate samples (50 µl) are removed from the incubation mixture at 0, 5, 10, 20, 40 and 60 min (control sample at 60 min only) and added to methanol, containing internal standard (100 µl), to stop the reaction.
Tolbutamide, 7-hydroxycoumarin, and testosterone are used as control compounds.
The samples are centrifuged (2500 rpm at 4°C for 20 min) and the supernatants at each time point are pooled for cassette analysis by LC-MS/MS using generic methods.

**Data Analysis**

From a plot of In peak area ratio (compound peak area/internal standard peak area) against time, the gradient of the line is determined. Subsequently, half-life and intrinsic clearance are calculated using the equations below:

$$\text{Elimination rate constant (k)} = (\text{- gradient})$$

$$\text{Half life } (t_{1/2})(\text{min}) = \frac{0.693}{k}$$

$$\text{Intrinsic Clearance } (CL_{int})(\mu l/\text{min/million cells}) = \frac{V \times 0.693}{t_{1/2}}$$

where V = Incubation volume (μl)/number of cells

### 3. Caco-2 Permeability (Bi-directional)

#### Experimental Procedure

Caco-2 cells obtained from the ATCC at passage number 27 are used. Cells (passage number 40-60) are seeded on to Millipore Multiscreen Caco-2 plates at 1 x 105 cells/$cm^2$. They are cultured for 20 days in DMEM and media is changed every two or three days. On day 20 the permeability study is performed.
Hanks Balanced Salt Solution (HBSS) pH7.4 buffer with 25 mM HEPES and 10 mM glucose at 37˚C is used as the medium in permeability studies. Incubations are carried out in an atmosphere of 5% $CO_2$ with a relative humidity of 95%. On day 20, the monolayers are prepared by rinsing both basolateral and apical surfaces twice with HBSS at 37˚C. Cells are then incubated with HBSS in both apical and basolateral compartments for 40 min to stabilize physiological parameters.
HBSS is then removed from the apical compartment and replaced with test compound dosing solutions. The solutions are made by diluting 10 mM test compound in DMSO with HBSS to give a final test compound concentration of 10 μM (final DMSO concentration 1 %). The fluorescent integrity marker lucifer yellow is also included in the dosing solution. Analytical standards are made from dosing solutions. Test compound permeability is assessed in duplicate. On each plate compounds of known permeability characteristics are run as controls.
The apical compartment inserts are then placed into 'companion' plates containing fresh HBSS. For basolateral to apical (B-A) permeability determination the experiment is initiated by replacing buffer in the inserts then placing them in companion plates containing dosing solutions. At 120 min the companion plate is removed and apical and basolateral samples diluted for analysis by LC-MS/MS. The starting concentration ($C_0$) and experimental recovery is calculated from both apical and basolateral compartment concentrations.
The integrity of the monolayers throughout the experiment is checked by monitoring lucifer yellow permeation using fluorimetric analysis. Lucifer yellow permeation is low if monolayers have not been damaged. Test and control compounds are quantified by LC-MS/MS cassette analysis using a 5-point calibration with appropriate dilution of the samples. Generic analytical conditions are used.
If a lucifer yellow $P_{app}$ value is above QC limits in one individual test compound well, then an n=1 result is reported. If lucifer yellow $P_{app}$ values are above QC limits in both replicate wells for a test compound, the compound is re-tested. Consistently high lucifer yellow permeation for a particular compound in both wells indicates toxicity. No further experiments are performed in this case.

#### Data Analysis

The permeability coefficient for each compound ($P_{app}$) is calculated from the following equation:

$$P_{app} = \frac{dQ/dt}{C_0 \times A}$$

Where dQ/dt is the rate of permeation of the drug across the cells, $C_0$ is the donor compartment concentration at time zero and A is the area of the cell monolayer. $C_0$ is obtained from analysis of donor and receiver compartments

at the end of the incubation period. It is assumed that all of the test compound measured after 120 min incubation was initially present in the donor compartment at 0 min. An asymmetry index (AI) is derived as follows:

$$AI = \frac{P_{app}\ (B\text{-}A)}{P_{app}\ (A\text{-}B)}$$

An asymmetry index above unity shows efflux from the Caco-2 cells, which indicates that the compound may have potential absorption problems in vivo.

The apparent permeability ($P_{app}$ (A-B)) values of test compounds are compared to those of control compounds, atenolol and propranolol, that have human absorption of approximately 50 and 90% respectively (Zhao, Y.H., et al., (2001). Evaluation of Human Intestinal Absorption Data and Subsequent Derivation of a Quantitative Structure-Activity Relationship (QSAR) with the Abraham Descriptors. Journal of Pharmaceutical Sciences. 90 (6), 749-784). Talinolol (a known P-gp substrate (Deferme, S., Mols, R., Van Driessche, W., Augustijns, P. (2002). Apricot Extract Inhibits the P-gp-Mediated Efflux of Talinolol. Journal of Pharmaceutical Sciences. 91 (12), 2539-48)) is also included as a control compound to assess whether functional P-gp is present in the Caco-2 cell monolayer.

**4. Cytochrome P450 Inhibition (5 Isoform $IC_{50}$ Determination))**

**Experimental Procedure**

**CYP1 A Inhibition**

Six test compound concentrations (0.05, 0.25, 0.5, 2.5, 5, 25 μM in DMSO; final DMSO concentration = 0.35%) are incubated with human liver microsomes (0.25 mg/ml) and NADPH (1 mM) in the presence of the probe substrate ethoxyresorufin (0.5 μM) for 5 min at 37°C. The selective CYP1A inhibitor, alpha-naphthoflavone, is screened alongside the test compounds as a positive control.

**CYP2C9 Inhibition**

Six test compound concentrations (0.05, 0.25, 0.5, 2.5, 5, 25 μM in DMSO; final DMSO concentration = 0.25%) are incubated with human liver microsomes (1 mg/ml) and NADPH (1 mM) in the presence of the probe substrate tolbutamide (120 μM) for 60 min at 37°C. The selective CYP2C9 inhibitor, sulphaphenazole, is screened alongside the test compounds as a positive control.

**CYP2C19 Inhibition**

Six test compound concentrations (0.05, 0.25, 0.5, 2.5, 5, 25 μM in DMSO; final DMSO concentration = 0.25%) are incubated with human liver microsomes (0.5 mg/ml) and NADPH (1 mM) in the presence of the probe substrate mephenytoin (25 μM) for 60 min at 37°C. The selective CYP2C19 inhibitor, tranylcypromine, is screened alongside the test compounds as a positive control.

**CYP2D6 Inhibition**

Six test compound concentrations (0.05, 0.25, 0.5, 2.5, 5, 25 μM in DMSO; final DMSO concentration = 0.25%) are incubated with human liver microsomes (0.5 mg/ml) and NADPH (1 mM) in the presence of the probe substrate dextromethorphane (5 μM) for 30 min at 37°C. The selective CYP2D6 inhibitor, quinidine, is screened alongside the test compounds as a positive control.

**CYP3A4 Inhibition**

Six test compound concentrations (0.05, 0.25, 0.5, 2.5, 5, 25 μM in DMSO; final DMSO concentration 0.26%) are incubated with human liver microsomes (0.25 mg/ml) and NADPH (1 mM) in the presence of the probe substrate midazolam (2.5 μM) for 5 min at 37°C. The selective CYP3A4 inhibitor, ketoconazole, is screened alongside the test compounds as a positive control.

For the CYP1A incubations, the reactions are terminated by the addition of methanol, and the formation of the

metabolite, resorufin, is monitored by fluorescence (excitation wavelength = 535 nm, emission wavelength = 595 nm). For the CYP2C9, CYP2C19, CYP2D6, and CYP3A4 incubations, the reactions are terminated by the addition of methanol containing internal standard. The samples are then centrifuged, and the supernatants are combined, for the simultaneous analysis of 4-hydroxytolbutamide, 4-hydroxymephenytoin, dextrorphan, and 1-hydroxymidazolam plus internal standard by LC-MS/MS. Generic LC-MS/MS conditions are used. Formic acid in deionised water (final concentration = 0.1 %) is added to the final sample prior to analysis. A decrease in the formation of the metabolites compared to vehicle control is used to calculate an $IC_{50}$ value (test compound concentration which produces 50% inhibition).

**5. Plasma Protein Binding (10%)**

**Experimental Procedure**

Solutions of test compound (5 μM, 0.5% final DMSO concentration) are prepared in buffer (pH 7.4) and 10% plasma (v/v in buffer). The experiment is performed using equilibrium dialysis with the two compartments separated by a semi-permeable membrane. The buffer solution is added to one side of the membrane and the plasma solution to the other side. Standards are prepared in plasma and buffer and are incubated at 37˚C. Corresponding solutions for each compound are analyzed in cassettes by LC-MS/MS.

**Quantitative Analysis**

After equilibration, samples are taken from both sides of the membrane. The solutions for each batch of compounds are combined into two groups (plasma-free and plasma-containing) then cassette analyzed by LC-MS/MS using two sets of calibration standards for plasma-free (7 points) and plasma-containing solutions (6 points). Generic LC-MS/MS conditions are used. Samples are quantified using standard curves prepared in the equivalent matrix. The compounds are tested in duplicate.
A control compound is included in each experiment.

**Data Analysis**

$$fu = \frac{1 - ((PC - PF))}{(PC)}$$

fu = fraction unbound
PC = sample concentration in protein containing side
PF = sample concentration in protein free side
fu at 10% plasma is converted to fu 100% plasma using the following equation:

$$fu_{100\%} = \frac{fu_{10\%}}{10-(9^{*}fu_{10\%})}$$

**Examples of a Pharmaceutical Composition**

As a specific embodiment of an oral composition of a compound of the present invention, 34 mg of Example 5 is formulated with sufficient finely divided lactose to provide a total amount of 580 to 590 mg to fill a size 0 hard gelatin capsule.

As another specific embodiment of an oral composition of a compound of the present invention, 35 mg of Example 11 is formulated with sufficient finely divided lactose to provide a total amount of 580 to 590 mg to fill a size 0 hard gelatin capsule.

While the invention has been described and illustrated in reference to certain preferred embodiments thereof, those skilled in the art will appreciate that various changes, modifications and substitutions can be made therein without

departing from the spirit and scope of the invention. For example, effective dosages, other than the preferred doses as set forth above, may be applicable as a consequence of the specific pharmacological responses observed and may vary depending upon the particular active compound selected, as well as from the type of formulation and mode of administration employed, and such expected variations or differences in the results are contemplated in accordance with the objects and practices of the present invention. It is intended, therefore, that the invention be limited only by the scope of the claims which follow and that such claims be interpreted as broadly as is reasonable.

## Claims

**1.** A compound according to formula (I)

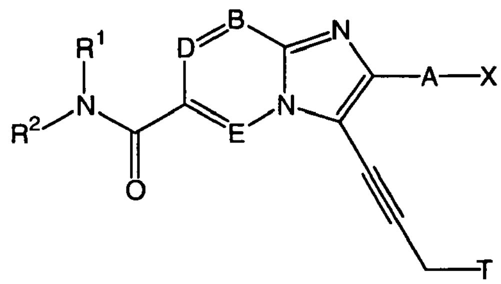

(I)

and enantiomers, diastereomers, tautomers, solvates and pharmaceutically acceptable salts thereof, wherein
$R^1$ and $R^2$ are independently from each other selected from

H,
$C_{1-6}$ alkyl,
$C_{1-6}$ alkylene-O-$C_{1-6}$alkyl
$C_{1-3}$alkylene-heterocyclyl, and
$C_{1-6}$ alkylene-$C_{3-7}$cycloalkyl, or

$R^1$ and $R^2$, together with the nitrogen atom to which they are attached to, form a 5 to 6-membered ring which may additionally contain one oxygen atom in the ring and which is unsubstituted or substituted by one or more substituents selected from OH,
$C_{1-6}$alkyl, O-$C_{1-6}$alkyl, $C_{0-3}$alkylene-$C_{3-5}$Cycloalkyl, $C_{1-6}$alkylene-O-$C_{1-6}$alkyl and $(CH_2)_{0-3}$ -phenyl;
B, D and E are independently from each other selected from CH and N;
A is -NH-,

-N($C_{1-6}$alkyl)-,
-$C_{1-6}$alkylene,
-$C_{2-6}$alkenylene,
-$C_{2-6}$alkinylene, or
a bond
wherein alkylene, alkenylene and alkinylene are unsubstituted or substituted with one ore more $R^6$;

$R^6$ is independently selected from

$C_{1-6}$alkyl,
$OR^{13}$,
$NR^{14a}R^{14b}$,
halogen,
phenyl, and
heteroaryl,
wherein phenyl and heteroaryl are unsubstituted or substituted by 1 to 3 $R^{3a}$;

X is H,

CN,
$C_{3-8}$cycloalkyl, unsubstituted or substituted with one or more halogen atoms, phenyl,
phenyl which is fused with

(a) a saturated or unsaturated heterocyclic 5 to 6-membered ring containing 1 or 2 heteroatoms independently selected from O and N, or
(b) a 5 to 6-membered heteroaryl containing 1 to 3 heteroatoms independently selected from O and N,

4 to 8-membered saturated or unsaturated heterocyclyl containing 1 to 4 heteroatoms independently selected from N, O and S,
5- to 6-membered heteroaryl containing 1 to 4 heteroatoms independently selected from N, O and S,
5- to 6-membered heteroaryl containing 1 to 4 heteroatoms independently selected from N, O and S, fused with

(a) 5 to 6-membered heteroaryl containing 1 to 4 heteroatoms independently selected from N, O and S
(b) a saturated or unsaturated heterocyclic 5 to 6-membered ring containing1 or 2 heteroatoms independently selected from O and N,

$-C(O)-R^5$,
$-OR^{13}$,
halogen or
$NR^{14a}R^{14b}$,
wherein each phenyl, heterocyclyl or heteroaryl is unsubstituted or substituted by 1 to 3 $R^{3a}$ and/or 1 $R^{3b}$ and/or 1 $R^4$;

$R^{3a}$ is independently selected from

halogen,
CN,
$C_{1-6}$alkyl, unsubstituted or substituted with one or more substituents independently selected from halogen, OH, $O-C_{1-6}$alkyl and 5 to 6-membered heterocyclyl containing 1 to 2 heteroatoms independently selected from N, O and S,
$O-C_{1-6}$alkyl, wherein alkyl is unsubstituted or substituted with one or more halogen atoms, and
OH;

$R^{3b}$ is $C(O)-C_{1-6}$alkyl,

$C(O)O-C_{1-6}$alkyl,
$C(O)NH_2$,
$C(O)NH-C_{1-6}$alkyl,
$C(O)N-(C_{1-6}alkyl)_2$,
$SO_2-C_{1-6}$alkyl,
$C(O)NH-SO_2-C_{1-6}$alkyl,
oxo, whereby the ring is at least partially saturated,
$NH_2$,
$NH-C_{1-6}$alkyl, wherein alkyl is unsubstituted or substituted by OH or
$O-C_{1-6}$ alkyl,
$N-(C_{1-6}alkyl)_2$, wherein each alkyl is unsubstituted or substituted by OH or $O-C_{1-6}$ alkyl,
$NH-SO_2-CH_3$, or
$NH-SO_2-CF_3$;

$R^4$ is 5 to 6-membered saturated or unsaturated heterocyclyl containing 1 to 3 heteroatoms independently selected from N, O and S, or

5 to 6-membered heteroaryl containing 1 to 3 heteroatoms independently selected from N, O and S,
wherein each heterocyclyl and heteroaryl is unsubstituted or substituted by 1 or 2 $R^{3a}$;

$R^5$ is H,

OH,
$C_{1-6}$alkyl, unsubstituted or substituted with one or more halogen atoms, O-$C_{1-6}$alky), wherein alkyl is unsubstituted or substituted with one or more
$R^{15}$,
phenyl,
4 to 8-membered saturated or unsaturated heterocyclyl containing 1 to 3 heteroatoms selected from N, O or S; or
$NR^{15a}R^{15b}$,
wherein each phenyl and heterocyclyl is unsubstituted or substituted by 1 to 3 $R^{3a}$ and/or 1 $R^4$;

T is

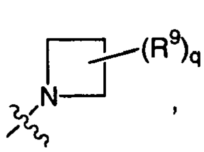 , 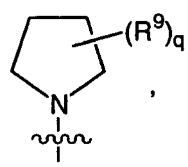 , 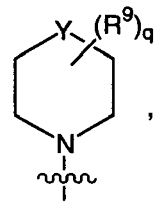 ,

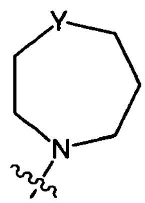 , 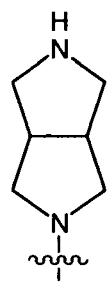 , 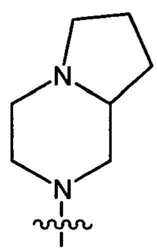 ,

or $NR^{11}R^{12}$;

$R^9$ is $NH_2$,

OH,
O-$C_{1-6}$alkyl, wherein alkyl is unsubstituted or substituted by 1 to 3 halogen atoms,
$C_{1-6}$alkyl, wherein alkyl is unsubstituted or substituted by 1 to 3 halogen atoms,
halogen,
NH($C_{1-6}$alkyl),
N($C_{1-6}$alkyl)$_2$,
phenyl, or
heteroaryl,
wherein phenyl and heteroaryl are unsubstituted or substituted by 1 to 3 $R^{3a}$;

q is 0, 1 or 2;
Y is $CH_2$,

$NR^{10}$, or
O;

$R^{10}$ is H,

$C_{1-6}$alkyl, or
$(CH_2)_{0-6}$-$C_{3-7}$cycloalkyl;

$R^{11}$ and $R^{12}$ are independently from each other selected from

H,
$C_{1-6}$ alkyl,
$(CH_2)_{0-2}$-$C_{3-7}$cycloalkyl and
$C_{1-6}$alkylene-O-$C_{1-6}$alkyl,
wherein alkyl, alkylene and cycloalkyl are unsubstituted or substituted by 1 to 3 $R^{3a}$;

$R^{13}$is H ,

$C_{1-6}$alkyl, unsubstituted or substituted with one or more substituents selected from halogen,
phenyl, or
heteroaryl,
wherein phenyl and heteroaryl are unsubstituted or substituted by 1 to 3 $R^{3a}$;

$R^{14a}$ and $R^{14b}$ are independently from each other selected from

H,
$C_{1-6}$alkyl, unsubstituted or substituted with one or more substituents selected from halogen, OH, O($C_{1-6}$alkyl), $NH_2$, NH($C_{1-6}$alkyl) and N($C_{1-6}$ alkyl)$_2$,
phenyl,
5 to 6-membered heteroaryl containing 1 to 4 heteroatoms independently selected from N, O and S,
wherein phenyl and heteroaryl are unsubstituted or substituted by 1 to 3 $R^{3a}$, and
C(O)$C_{1-6}$alkyl;

$R^{15}$, $R^{15a}$ and $R^{15b}$ are independently from each other selected from

H,
$C_{1-6}$alkyl, unsubstituted or substituted with one or more substituents selected from halogen, OH, O($C_{1-6}$alkyl), $NH_2$, NH($C_{1-6}$alkyl) and N($C_{1-6}$ alkyl)$_2$,
$C_{0-3}$alkylene-$C_{3-5}$Cycloalkyl,
phenyl and
5 to 6-membered heteroaryl containing 1 to 4 heteroatoms independently selected from N, O and S,
wherein phenyl and heteroaryl are unsubstituted or substituted by 1 to 3 $R^{3a}$.

**2.** The compound of claim 1, wherein B, D and E are simultaneously CH.

**3.** The compound of claim 1 or 2 wherein A is -NH-, $C_{2-6}$alkinylene or a bond.

**4.** The compound of any of claims 1 to 3 wherein $R^1$ and $R^2$ are independently from each other $C_{3-6}$alkyl, $C_{1-6}$alkylene-$C_{3-7}$cycloalkyl or $R^1$ and $R^2$ form together with the nitrogen atom to which they are attached to a 5 to 6-membered ring which may additionally contain one oxygen atom in the ring and which is unsubstituted or substituted by one or more substituents selected from OH, $C_{1-6}$alkyl, $C_{0-3}$alkylene-$C_{3-5}$cycloalkyl, O-$C_{1-6}$alkyl, $C_{1-6}$alkylene-O-$C_{1-6}$alkyl and $(CH_2)_{0-3}$-phenyl.

**5.** The compound of any of claims 1 to 4 wherein T is selected from

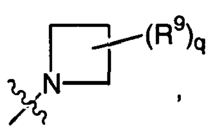 , 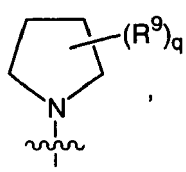 , 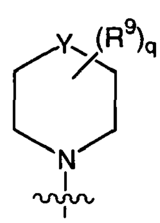

or

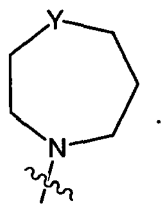

6. The compound of claim 5 wherein Y is $CH_2$ or $NR^{10}$, $R^9$ is $NH_2$, $C_{1-6}$alkyl, $NH(C_{1-6}alkyl)$ or $N(C_{1-6}a)ky))_2$ and q is 0 or 1.

7. The compound of any of claims 1 to 6, wherein
X is phenyl,

phenyl which is fused with

(a) a saturated or unsaturated heterocyclic 5 to 6-membered ring, containing 1 or 2 heteroatoms independently selected from O and N, or
(b) a 5 to 6-membered heteroaryl containing 1 to 3 heteroatoms independently selected from O and N,

4 to 8-membered saturated or unsaturated heterocyclyl containing 1 to 4 heteroatoms independently selected from N, O and S, or
5 to 6-membered heteroaryl containing 1 to 4 heteroatoms independently selected from N, O and S,
5 to 6-membered heteroaryl containing 1 to 4 heteroatoms independently selected from N, O and S, fused with

(a) 5 to 6-membered heteroaryl containing 1 to 4 heteroatoms independently selected from N, O and S,
(b) a saturated or unsaturated heterocyclic 5- or 6-membered ring containing 1 or 2 heteroatoms independently selected from O and N,

wherein each phenyl, heterocyclyl or heteroaryl is unsubstituted or substituted by 1 to 3 $R^{3a}$ and/or 1 $R^{3b}$ and/or 1 $R^4$, or
-C(O)-$R^5$.

8. The compound of claim 7, wherein $R^5$ is -O-$C_{1-6}$alkyl, wherein alkyl is unsubstituted or substituted with one or more $R^{15}$.

9. The compound of any of claims 1 to 8 as a medicament.

10. The compound of any of claims 1 to 8 as a melanocortin-4 receptor antagonist.

11. The compound of any of claims 1 to 8 for use in the prophylaxis or treatment of disorders, diseases or conditions responsive to the inactivation of the melanocortin-4 receptor in a mammal.

12. The compound of claim 11 for use in the prophylaxis or treatment of cancer cachexia, muscle wasting, anorexia, anxiety and/or depression, emesis or amyotrophic lateral sclerosis (ALS).

13. Use of the compound of any of claims 1 to 8 for the preparation of a medicament for the prophylaxis or treatment of disorders, diseases or conditions responsive to the inactivation of the melanocortin-4 receptor in a mammal.

14. A pharmaceutical composition comprising a compound of any of claims 1 to 8 and a pharmaceutically acceptable carrier.

Europäisches Patentamt
European Patent Office
Office européen des brevets

# EUROPEAN SEARCH REPORT

Application Number
EP 08 01 6887

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,A | WO 02/066478 A (ASTRAZENECA AB [SE]; ASTRAZENECA UK LTD [GB]; DOSSETTER ALEXANDER GRAH) 29 August 2002 (2002-08-29) claim 1, examples K1 or K2 in pages 63 and 65, and example B1 in page 45 ----- | 1-14 | INV. C07D471/04 A61K31/437 A61P3/00 |
| A | WO 2004/089951 A (SOD CONSEILS RECH APPLIC [FR]; POITOUT LYDIE [FR]; BRAULT VALERIE [FR]) 21 October 2004 (2004-10-21) claim 1, abstract ----- | 1-14 | |
| A | POITOUT ET AL: "Identification of a novel series of benzimidazoles as potent and selective antagonists of the human melanocortin-4 receptor" BIOORGANIC & MEDICINAL CHEMISTRY LETTERS, PERGAMON, ELSEVIER SCIENCE, GB, vol. 17, no. 16, 17 July 2007 (2007-07-17), pages 4464-4470, XP022181848 ISSN: 0960-894X whole document, specially compounds 4a-3, 5b-p, 6a-z, 11a-h and 17 ----- | 1-14 | TECHNICAL FIELDS SEARCHED (IPC): C07D |

The present search report has been drawn up for all claims

2

| Place of search | Date of completion of the search | Examiner |
|---|---|---|
| Munich | 24 February 2009 | Sahagún Krause, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 08 01 6887

This annex lists the patent family members relating to the patent documents cited in the above-mentioned European search report.
The members are as contained in the European Patent Office EDP file on
The European Patent Office is in no way liable for these particulars which are merely given for the purpose of information.

24-02-2009

| Patent document cited in search report | Publication date | Patent family member(s) | Publication date |
|---|---|---|---|
| WO 02066478 A | 29-08-2002 | NONE | |
| WO 2004089951 A | 21-10-2004 | AU 2004228416 A1 | 21-10-2004 |
| | | BR PI0408817 A | 04-04-2006 |
| | | CA 2520855 A1 | 21-10-2004 |
| | | CN 1768058 A | 03-05-2006 |
| | | EP 1615925 A1 | 18-01-2006 |
| | | FR 2852957 A1 | 01-10-2004 |
| | | JP 2006522076 T | 28-09-2006 |
| | | KR 20060002893 A | 09-01-2006 |
| | | MX PA05010278 A | 17-11-2005 |
| | | NZ 542763 A | 30-11-2007 |
| | | US 2006173036 A1 | 03-08-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0074679 A
- WO 02066478 A


### Non-patent literature cited in the description


- **A. Kask et al.** Selective antagonist for the melanocortin-4 receptor (HS014) increases food intake in free-feeding rats. *Biochem. Biophys. Res. Commun.,* 1998, vol. 245, 90-93
- **Chen et al.** *Cell,* 1997, vol. 91, 789-798
- **S.Q. Giraudo et al.** Feeding effects of hypothalamic injection of melanocortin-4 receptor ligands. *Brain Research,* 1998, vol. 80, 302-306
- **D. Huszar et al.** *Cell,* 1997, vol. 88, 131-141
- **I. Corcos et al.** HP-228 is a potent agonist of melanocortin receptor-4 and significantly attenuates obesity and diabetes in Zucker fatty rats. *Society for Neuroscience Abstracts,* 1997, vol. 23, 673
- **H. Wessells et al.** Synthetic Melanotropic Peptide Initiates Erections in Men With Psychogenic Erectile Dysfunction: Double-Blind, Placebo Controlled Crossover Study. *J. Urol.,* 1998, vol. 160, 389-393
- **Owen MJ ; Nemeroff CB.** Physiology and pharmacology of corticotrophin releasing factor. *Pharmacol Rev,* 1991, vol. 43, 425-473
- **Shigeyuki Chaki et al.** *J. Pharm. Exp. Ther.,* 2003, vol. 304 (2), 818-26
- **Marks D.L. et al.** Role of the central melanocortin system in cachexia. *Cancer Res.,* 2001, vol. 61, 1432-1438
- **Ludolph AC.** *Neuromuscul Disord.,* 2006, vol. 16 (8), 530-8
- **Herrmann, W.A. et al.** *Angew. Chem.,* 1995, vol. 107, 1989-1992
- **Biel ; DiPierro.** *J. Am. Chem. Soc.,* 1958, vol. 80, 4609-4614
- **Chen, A.S. et al.** *Transgenic Res,* April 2000, vol. 9 (2), 145-54
- **Marks, D.L. ; Ling, N ; Cone, R.D.** *Cancer Res,* 15 February 2001, vol. 61 (4), 1432-8
- **Zhao, Y.H. et al.** Evaluation of Human Intestinal Absorption Data and Subsequent Derivation of a Quantitative Structure-Activity Relationship (QSAR) with the Abraham Descriptors. *Journal of Pharmaceutical Sciences,* 2001, vol. 90 (6), 749-784
- **Deferme, S. ; Mols, R. ; Van Driessche, W. ; Augustijns, P.** Apricot Extract Inhibits the P-gp-Mediated Efflux of Talinolol. *Journal of Pharmaceutical Sciences,* 2002, vol. 91 (12), 2539-48